(19) Europäisches Patentamt
European Patent Office
Office européen des brevets

(11) EP 3 689 952 B1

# (12) EUROPEAN PATENT SPECIFICATION

(45) Date of publication and mention of the grant of the patent:
**20.07.2022 Bulletin 2022/29**

(21) Application number: **19220030.1**

(22) Date of filing: **30.12.2019**

(51) International Patent Classification (IPC):
***C08J 7/04*** *(2020.01)* ***A61M 5/315*** *(2006.01)*

(52) Cooperative Patent Classification (CPC):
**C08J 7/04; A61M 5/31513;** A61M 2205/0222; C08J 2327/18; C08J 2333/08

## (54) SYRINGE GASKET

SPRITZENDICHTUNG

JOINT DE SERINGUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.01.2019 JP 2019014469**

(43) Date of publication of application:
**05.08.2020 Bulletin 2020/32**

(73) Proprietor: **Sumitomo Rubber Industries, Ltd.**
**Kobe-shi, Hyogo-ken 651-0072 (JP)**

(72) Inventors:
- **MINAGAWA, Yasuhisa**
  **Kobe-shi, Hyogo 651-0072 (JP)**
- **FUJIMOTO, Kentaro**
  **Kobe-shi, Hyogo 651-0072 (JP)**

(74) Representative: **Manitz Finsterwald**
**Patent- und Rechtsanwaltspartnerschaft mbB**
**Martin-Greif-Strasse 1**
**80336 München (DE)**

(56) References cited:
**EP-A1- 3 281 661**
**EP-A1- 3 553 116**
**EP-A1- 3 639 863**
**JP-A- 2015 043 827**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 689 952 B1

## Description

BACKGROUND OF THE INVENTION

Field of the Invention

The present invention relates to a syringe gasket.

Description of the Background Art

In view of the importance of sealability, elastic bodies such as rubber are used for a portion that slides while maintaining a sealing state thereof, for example, for a gasket that is integrated with the plunger of an injector and seals the syringe together with the plunger. However, such elastic bodies have a slight problem in slidability (see Japanese Laid-Open Patent Publication No. 2004-298220). Thus, a slidability improver such as silicone oil is applied to a sliding surface, but a possibility of silicone oil having an adverse effect on recently marketed biopharmaceuticals has been pointed out. Meanwhile, a gasket having no slidability improver applied thereon has inferior slidability, and thus, during administration, does not allow a plunger to be smoothly pressed, thus resulting in pulsation occurring, causing problems such as the injection volume becoming inaccurate and giving pain to the patient.

Moreover, for such a syringe gasket, a crosslinked rubber to which various additives for crosslinking are added is generally used, and the additives or thermal decomposition products thereof may be transferred into a drug solution when the additives or the thermal decomposition products come into contact with the drug solution, although the amount is very small. Thus, there is also a problem that the transferred additives and the like adversely affect the effect and stability of the drug solution.

As described above, syringe gaskets have been required to ensure sealability (liquid leakage resistance) and slidability, which have a trade-off relationship, as well as chemical resistance, protein adsorption inhibition performance, etc., and various studies have been made for the requirements. For example, a technology for coating with a PTFE film that has self lubricity has been proposed (see Japanese Laid-Open Patent Publication No. 2010-142537), but such a film is generally expensive, so that the production cost of a processed product increases, resulting in a limited application range. Moreover, when a product coated with a PTFE film is applied to applications where durability is required due to repeated sliding or the like, there is also a concern about reliability. JP 2015/043827 A discloses a syringe gasket having an inert film on its liquid-contacting part and a polymer chain on the surface of its sliding part. EP 3 281 661 A1 discloses a syringe gasket having on its surface an acrylic acid-acrylamide copolymer chain modified by adding a tetraalkoxysilane and reacting with a perfluoroethercontaining silane. EP 3 553 116 A1, which has been published after the priority date of the present application, discloses a syringe gasket having on its surface an acrylic acid-acrylamide copolymer chain modified by adding a tetraalkoxysilane and reacting with a fluoroalkyl silane and a perfluoroether silane. EP 3 639 863 A1, which has been published after the filing date of the present application, discloses a syringe gasket, the rubber surface of which has been modified by adding a tetraalkoxysilane and reacting with a fluoroalkyl silane and a perfluoroether silane.

The present invention has been made to solve the above-described problem, and an object of the present invention is to provide a syringe gasket having excellent performance such as slidability, liquid leakage resistance, and protein adsorption inhibition performance.

SUMMARY OF THE INVENTION

The present invention is directed to a syringe gasket including a liquid contact portion and a sliding portion, wherein an inactive film is layered on the liquid contact portion, no inactive film is layered on the sliding portion, a modified polymer chain is formed on a surface of the sliding portion, the modified polymer chain is obtained by adding a silane compound to a surface of a polymer chain and further reacting a fluoroalkyl group-containing silane compound and a perfluoroether group-containing silane compound or further reacting a fluoroalkyl group-containing silane compound, a mass ratio of the fluoroalkyl group-containing silane compound to the perfluoroether group-containing silane compound is 60:40 to 100:0, and the fluoroalkyl group-containing silane compound is a compound represented by the following formula (1),

$$F_3C\text{-}(CF_2)_n\text{-}(CH_2)_m\text{-}Si(OR^1)_3 \qquad (1)$$

wherein n is 0 to 5, m is 0 to 8, and $R^1$s each represent an alkyl group and may be the same or different from each other. In the following the expression further reacting the fluoroalkyl group-containing silane compound and/or the perfluoroether group-containing silane compound is to be interpreted as further reacting the fluoroalkyl group-containing silane compound and the perfluoroether group-containing silane compound or further reacting the fluoroalkyl group-containing silane compound.

The inactive film is preferably formed from an olefin-based resin and/or at least one fluorine resin selected from the group consisting of a tetrafluoroethylene polymer, a tetrafluoroethylene-ethylene copolymer, a modified tetrafluoroethylene polymer, a modified tetrafluoroethylene-ethylene copolymer, and a chlorofluoroethylene polymer.

The modified polymer chain is preferably formed by a formation method including: a step 1 of forming a polymerization initiation point on the surface of the sliding portion; a step 2 of radically polymerizing a monomer starting from the polymerization initiation point to grow a polymer chain on the surface of the sliding portion; and a step 3 of adding the silane compound to a surface of the polymer chain and further reacting the fluoroalkyl group-containing silane compound and/or the perfluoroether group-containing silane compound to form the modified polymer chain.

The modified polymer chain is preferably formed by a formation method including: a step I of radically polymerizing a monomer in the presence of a photopolymerization initiator to grow a polymer chain on the surface of the sliding portion; and a step II of adding the silane compound to a surface of the polymer chain and further reacting the fluoroalkyl group-containing silane compound and/or the perfluoroether group-containing silane compound to form the modified polymer chain.

The monomer is preferably at least one monomer selected from the group consisting of acrylic acid, acrylic acid esters, alkali metal salts of acrylic acid, amine salts of acrylic acid, acrylamide, dimethylacrylamide, diethylacrylamide, isopropylacrylamide, hydroxyethylacrylamide, acryloylmorpholine, methoxymethylacrylate, hydroxyethylacrylate, methacrylic acid, methacrylic acid esters, alkali metal salts of methacrylic acid, amine salts of methacrylic acid, methacrylamide, dimethylmethacrylamide, diethylmethacrylamide, isopropylmethacrylamide, hydroxyethylmethacrylamide, methacryloylmorpholine, methoxymethyl methacrylate, hydroxyethyl methacrylate, and acrylonitrile.

The fluoroalkyl group is a group represented by the following formula,

[Chem. 1] $F_3C\text{-}(CF_2)_n\text{-}(CH_2)_m\text{-}$

wherein n is an integer from 0 to 5, and m is an integer from 0 to 8.

The fluoroalkyl group-containing silane compound is a compound represented by the following formula (1),

[Chem. 2] $F_3C\text{-}(CF_2)_n\text{-}(CH_2)_m\text{-}Si(OR^1)_3$ (1)

wherein n is 0 to 5, m is 0 to 8, and $R^1$s each represent an alkyl group and may be the same or different from each other.

The perfluoroether group-containing silane compound is preferably a compound represented by the following formula (2) or (3),

[Chem. 3]

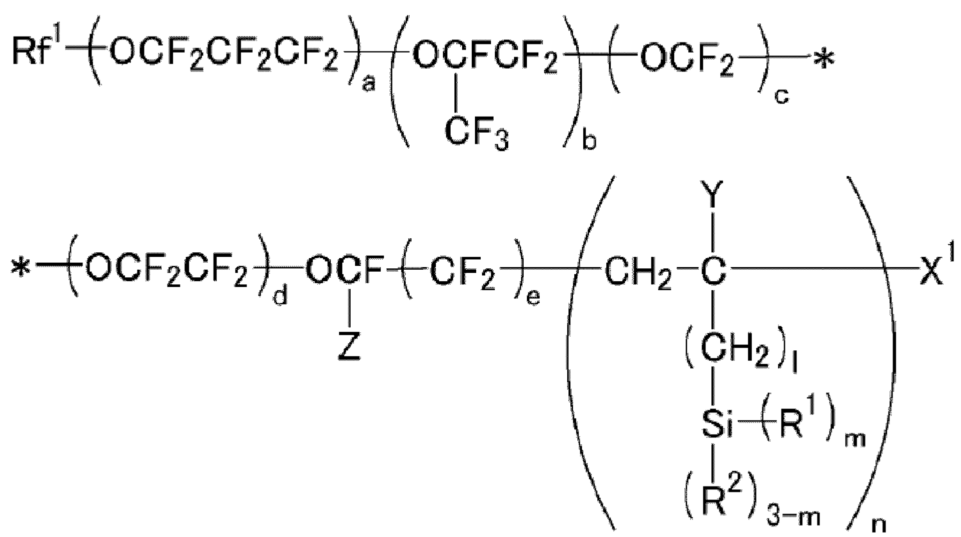

(2)

wherein $Rf^1$ represents a perfluoroalkyl group; Z represents fluorine or a trifluoromethyl group; a, b, c, d, and e are the same or different from each other and each represent an integer of 0 or 1 or more, a+b+c+d+e is 1 or more, the existing order of repeating units parenthesized by subscripts a, b, c, d, and e is not limited to that shown in the formula; Y

represents hydrogen or a C1-C4 alkyl group; $X^1$ represents hydrogen, bromine, or iodine; $R^1$ represents a hydroxyl group or a hydrolyzable substituent; $R^2$ represents hydrogen or a monovalent hydrocarbon group; 1 represents 0, 1, or 2; m represents 1, 2, or 3; and n represents an integer of 1 or more, and the two ends marked by * are directly bonded to each other,

[Chem. 4]

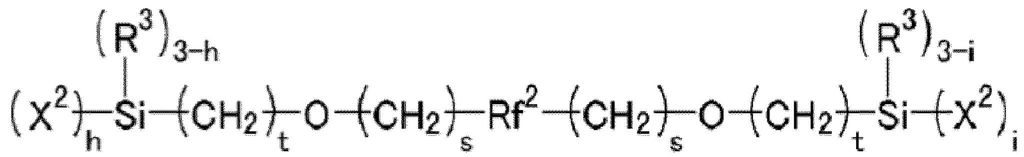 (3)

wherein $Rf^2$ represents a divalent group that contains a unit represented by $-(C_kF_{2k})O-$ (k is an integer from 1 to 6) and that has a non-branched linear perfluoropolyalkylene ether structure; $R^3$s are the same or different from each other and each represent a C1-C8 monovalent hydrocarbon group; $X^2$s are the same or different from each other and each represent a hydrolyzable group or a halogen atom; each s is the same or different from each other and represents an integer from 0 to 2; each t is the same or different from each other and represents an integer from 1 to 5; and h and i are the same or different from each other and each represent 1, 2, or 3.

The modified polymer chain preferably has a length of 200 to 7,000 nm.

Preferably, the modified polymer chain is fixed to at least a part of a surface of a base gasket, the sliding portion has a plurality of annular protrusions on a sliding surface thereof, and the base gasket has a surface roughness Ra equal to or less than 1.0 at a first protrusion closest to a top surface among the annular protrusions.

The surface roughness Ra of the base gasket is preferably equal to or less than 0.8.

The surface roughness Ra of the base gasket is preferably equal to or less than 0.6.

Preferably, the sliding portion has a plurality of annular protrusions on a sliding surface thereof, and a first protrusion closest to a top surface among the annular protrusions has a surface roughness Ra equal to or less than 1.0.

The surface roughness Ra is preferably equal to or less than 0.8.

The surface roughness Ra is preferably equal to or less than 0.6.

Preferably, the sliding portion has a plurality of annular protrusions on a sliding surface thereof, and a first protrusion closest to a top surface among the annular protrusions has a surface roughness Rz equal to or less than 25.0.

Preferably, the sliding portion has a plurality of annular protrusions on a sliding surface thereof, and a first protrusion closest to a top surface among the annular protrusions has a surface roughness Rv equal to or less than 21.0.

According to the present invention, the syringe gasket includes a liquid contact portion and a sliding portion, an inactive film is layered on the liquid contact portion, no inactive film is layered on the sliding portion, a modified polymer chain is formed on a surface of the sliding portion, the modified polymer chain is obtained by adding a silane compound to a surface of a polymer chain and further reacting a fluoroalkyl group-containing silane compound and a perfluoroether group-containing silane compound, a mass ratio of the fluoroalkyl group-containing silane compound to the perfluoroether group-containing silane compound is 60:40 to 100:0, and the fluoroalkyl group-containing silane compound is a compound represented by the following formula (1),

$$F_3C-(CF_2)_n-(CH_2)_m-Si(OR^1)_3 \qquad (1)$$

wherein n is 0 to 5, m is 0 to 8, and $R^1$s each represent an alkyl group and may be the same or different from each other. Thus, it is possible to provide a syringe gasket having excellent performance such as slidability, liquid leakage resistance, and protein adsorption inhibition performance.

BRIEF DESCRIPTION OF THE DRAWINGS

FIG. 1 shows an example of a schematic cross-sectional view of a gasket (object to be modified) in which an inactive film is layered on a liquid contact portion of a base gasket and no inactive film is layered on a sliding portion of the base gasket.

DESCRIPTION OF THE PREFERRED EMBODIMENTS

The syringe gasket according to the present invention is a syringe gasket including a liquid contact portion and a sliding portion, wherein an inactive film is layered on the liquid contact portion, no inactive film is layered on the sliding

portion, a modified polymer chain is formed on a surface of the sliding portion, the modified polymer chain is obtained by adding a silane compound to a surface of a polymer chain and further reacting a fluoroalkyl group-containing silane compound and a perfluoroether group-containing silane compound, a mass ratio of the fluoroalkyl group-containing silane compound to the perfluoroether group-containing silane compound is 60:40 to 100:0, and the fluoroalkyl group-containing silane compound is a compound represented by the following formula (1),

$$F_3C\text{-}(CF_2)_n\text{-}(CH_2)_m\text{-}Si(OR^1)_3 \qquad (1)$$

wherein n is 0 to 5, m is 0 to 8, and $R^1$s each represent an alkyl group and may be the same or different from each other.

The present invention is a syringe gasket in which the fluoroalkyl group-containing silane compound and/or the perfluoroether group-containing silane compound is formed on an outermost surface at a predetermined ratio by forming a modified polymer chain obtained by forming a polymer chain with a monomer, adding the silane compound onto the polymer chain, and then further reacting (adding or the like) the fluoroalkyl group-containing silane compound and/or the perfluoroether group-containing silane compound at a predetermined ratio with the silane compound. Since the modified polymer chain having the fluoroalkyl group-containing silane compound and/or the perfluoroether group-containing silane compound, which have low surface free energy, formed on the outermost surface at the predetermined ratio is formed, excellent performance such as slidability, liquid leakage resistance, biocompatibility, and protein adsorption inhibition performance can be economically imparted to an object to be modified. Here, when the fluoroalkyl group-containing silane compound and/or the perfluoroether group-containing silane compound is merely formed at a predetermined ratio without forming a relatively inexpensive polymer chain, desired performance such as sufficient slidability is not achieved.

Hereinafter, an example of an embodiment of the present invention will be described.

The syringe gasket according to the present invention is not particularly limited as long as the syringe gasket has the above structure. The syringe gasket according to the present invention can be produced, for example, by a production method including: for a gasket (object to be modified) in which an inactive film is layered on a liquid contact portion of a base gasket and no inactive film is layered on a sliding portion of the base gasket, forming a polymer chain on the surface of the sliding portion of the object to be modified (the sliding portion of the base gasket on which no inactive film is formed); then adding the silane compound to the surface of the polymer chain; and further reacting (adding or the like) the fluoroalkyl group-containing silane compound and/or the perfluoroether group-containing silane compound at a predetermined ratio.

As the base gasket, for example, the prefilled syringe gasket disclosed in Japanese Laid-Open Patent Publication No. 2012-147859, etc., can be used, but the base gasket is not particularly limited thereto.

FIG. 1 shows an example of a schematic cross-sectional view of a gasket (object to be modified) in which an inactive film is layered on a liquid contact portion of a base gasket and no inactive film is layered on a sliding portion of the base gasket. An object 1 to be modified in FIG. 1 in which an inactive film 15 is layered on a base gasket 11 has a liquid contact portion 12 that comes into contact with a drug solution within a syringe and that does not come into contact with the inner wall of the syringe during sliding, a sliding portion 13 that comes into contact with the inner wall of the syringe during sliding, and a fitting hole 14 into which a plunger rod is fitted.

The inactive film 15 is layered on the liquid contact portion 12, and FIG. 1 shows an example of the liquid contact portion 12 having a projection. The inactive film 15 is layered on the liquid contact portion 12, and is not layered on the sliding portion 13. By disposing the inactive film 15 as described above, the chemical resistance of the object 1 to be modified is improved, and both sealability and slidability can also be achieved in the object 1 to be modified.

As the material of the base gasket 11, a vulcanized molded rubber or a molded thermoplastic elastomer is used. As the vulcanized rubber or the thermoplastic elastomer, a material having a carbon atom adjacent to a double bond (a carbon atom at an allylic position) is suitably used.

Examples of the rubber include diene-based rubbers such as styrene butadiene rubber, butadiene rubber, isoprene rubber, natural rubber, and deproteinized natural rubber, butyl rubber containing several percent of isoprene units as a degree of unsaturation, and halogenated butyl rubber (chlorobutyl rubber, bromobutyl rubber). In the case of butyl rubber or halogenated butyl rubber, a rubber crosslinked by triazine is preferable from the viewpoint of reducing the extract from the vulcanized rubber. In this case, an acid acceptor may be contained, and examples of suitable acid acceptors include hydrotalcite and magnesium carbonate.

Moreover, the rubber can also be vulcanized by sulfur (sulfur vulcanization). In this case, blending agents that are generally used for sulfur vulcanization, such as a vulcanization accelerator, zinc oxide, a filler, and a silane coupling agent, may be added. Carbon black, silica, clay, talc, calcium carbonate, etc., can be suitably used as the filler.

The vulcanization conditions for the rubber may be set as appropriate, and the vulcanization temperature for the rubber is preferably not lower than 150°C, more preferably not lower than 170°C, and further preferably not lower than 175°C.

Examples of the thermoplastic elastomer include: a polymer compound that has rubber elasticity at room

temperature by aggregates of plastic components (hard segments) serving as crosslinking points (for example, thermoplastic elastomers (TPEs) such as a styrenebutadiene styrene copolymer); and a polymer compound that has rubber elasticity and in which a thermoplastic component and a rubber component are mixed and dynamically crosslinked with each other by a crosslinking agent (for example, thermoplastic elastomers (TPVs) such as a polymer alloy containing a styrene-based block copolymer or an olefin-based resin and a crosslinked rubber component).

Moreover, examples of other suitable thermoplastic elastomers include nylon, polyester, urethane, polypropylene, and dynamically crosslinked thermoplastic elastomers thereof. In the case of a dynamically crosslinked thermoplastic elastomer, a thermoplastic elastomer in which halogenated butyl rubber is dynamically crosslinked is preferable. In this case, the thermoplastic elastomer is preferably nylon, urethane, polypropylene, an SIBS (styreneisobutylene-styrene block copolymer), or the like.

The inactive film is not particularly limited, and fluorine resins such as a tetrafluoroethylene polymer (PTFE), a tetrafluoroethylene-ethylene copolymer (ETFE), a modified tetrafluoroethylene polymer, a modified tetrafluoroethylene-ethylene copolymer, and a chlorofluoroethylene polymer (PCTFE); olefin-based resins; etc., are preferable. In addition, from the viewpoint of achieving good chemical resistance, PTFE, ETFE, PCTFE, and olefin-based resins are more preferable, and PTFE is particularly preferable.

Examples of olefin-based resins include: polyethylene-based resins such as polyethylene, ethylene-propylene copolymers, ethylene-propylene-nonconjugated diene copolymers, ethylene-butene copolymers, ethylene-hexene copolymers, ethylene-octene copolymers, ethylene-vinyl acetate copolymers, ethylene-vinyl alcohol copolymers, ethyleneethyl acrylate copolymers, and chlorinated polyethylene; polypropylene-based resins such as polypropylene, propylene-ethylene random copolymers, propylene-ethylene block copolymers, and chlorinated polypropylene; and copolymers of polybutene, polyisobutylene, polymethylpentene, and cyclic olefins. Polyethylene (especially, ultra high molecular weight polyethylene (UHMWPE)) is preferable. An olefin-based resin may contain fluorine.

The thickness of the inactive film 15 may be adjusted as appropriate in accordance with the shape or size of the gasket 1, and is preferably 30 to 200 $\mu$m.

The inactive film 15 is preferably subjected to a treatment that increases adhesion to rubber or the like. Examples of the treatment that increases adhesion include chemical treatment methods, treatments that roughen the surface of the film, and combinations of these treatments. Specific examples of the treatment that increases adhesion include sodium treatment, glow discharge treatment, plasma treatment (discharge treatment) under atmospheric pressure or under a vacuum, excimer laser treatment (discharge treatment), and ion beam treatment.

An angle a formed by an end portion of the liquid contact portion 12 and the sliding portion 13 is preferably 90 to 110 degrees. The angle a is preferably equal to or less than 108 degrees, more preferably equal to or less than 106 degrees, further preferably equal to or less than 105 degrees, and particularly preferably equal to or less than 104 degrees.

Four annular protrusions that come into sliding contact with the inner circumferential surface of the circumferential body part of a barrel, that is, a first protrusion 17a (first protrusion 17a at the endmost liquid contact portion side) located closest to the liquid contact portion 12, a bottom-side protrusion 17c (bottom-side protrusion 17c at the endmost bottom side) located farthest from the liquid contact portion 12, and intermediate protrusions 17b (two protrusions) located between the protrusions 17a and 17c, are provided on the outer circumferential surface of the sliding portion 13 of the base gasket 11. In the base gasket in FIG. 1, the liquid contact portion 12 and the first protrusion 17a are formed so as to be integrated with each other.

FIG. 1 shows an embodiment having four annular protrusions, and the number of protrusions is preferably equal to or greater than 2 and is not particularly limited. In addition,

FIG. 1 shows an embodiment having two intermediate protrusions 17b, all protrusions located between the first protrusion and the bottom-side protrusion correspond to intermediate protrusions, and one or three or more intermediate protrusions may be provided. From the viewpoint of achieving both slidability and liquid leakage resistance, the number of annular protrusions of the base gasket 11 is preferably equal to or greater than three.

In the syringe gasket, the polymer chain can be formed on the surface of the sliding portion, for example, by a formation method including: a step 1 of forming a polymerization initiation point on the surface of the sliding portion in the object to be modified (base gasket); a step 2 of radically polymerizing a monomer starting from the polymerization initiation point to grow a polymer chain on the surface of the sliding portion; and a step 3 of adding a silane compound to the surface of the polymer chain and further reacting a fluoroalkyl group-containing silane compound and/or a perfluoroether group-containing silane compound at a predetermined ratio to form a modified polymer chain.

In the step 1, the polymerization initiation point is formed on the surface of the sliding portion of the object to be modified (base gasket). The step 1 can be carried out, for example, by adsorbing a photopolymerization initiator onto the surface of the sliding portion to form the polymerization initiation point.

Examples of the photopolymerization initiator include carbonyl compounds, organic sulfur compounds such as tetraethylthiuram disulfide, persulfides, redox compounds, azo compounds, diazo compounds, halogen compounds, and photoreductive pigments. Among these initiators, carbonyl compounds are preferable.

The carbonyl compound as the photopolymerization initiator is preferably benzophenone or a derivative thereof

(benzophenone compound), and, for example, a benzophenone compound represented by the following formula can be suitably used.

[Chem. 5]

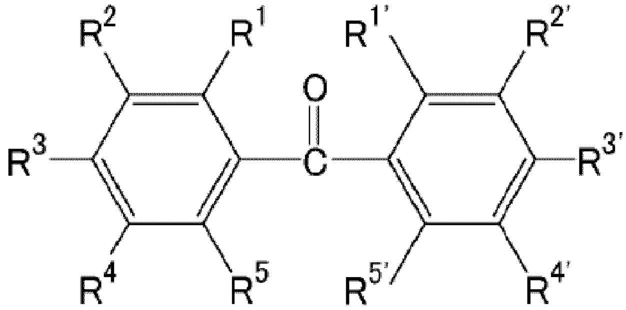

wherein $R^1$ to $R^5$ and $R^{1'}$ to $R^{5'}$ are the same or different from each other and each represent a hydrogen atom, an alkyl group, a halogen (fluorine, chlorine, bromine, or iodine), a hydroxyl group, a primary to tertiary amino group, a mercapto group, or a hydrocarbon group optionally containing an oxygen atom, a nitrogen atom, or a sulfur atom; and any two adjacent groups thereof may be joined to each other to form a cyclic structure together with the carbon atoms to which these two adjacent groups are bonded.

Specific examples of the benzophenone compound include benzophenone, xanthone, 9-fluorenone, 2,4-dichlorobenzophenone, methyl o-benzoylbenzoate, 4,4'-bis(dimethylamino)benzophenone, and 4,4'-bis(diethylamino)benzophenone. Among these compounds, benzophenone, xanthone, and 9-fluorenone are particularly preferable because good polymer brushes can be formed.

Fluorobenzophenone compounds can be suitably used as the benzophenone compound, and examples of fluorobenzophenone compounds include 2,3,4,5,6-pentafluoro benzophenone and decafluoro benzophenone.

Thioxanthone compounds can be suitably used as the photopolymerization initiator because the thioxanthone compounds provide a high polymerization rate and also can easily be adsorbed on and/or reacted with rubber or the like. For example, a compound represented by the following formula can be suitably used.

[Chem. 6]

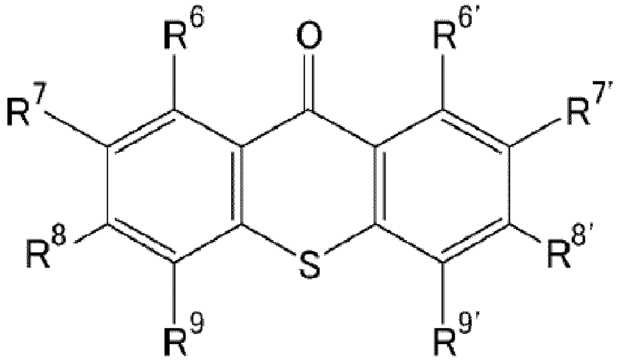

wherein $R^6$ to $R^9$ and $R^{6'}$ to $R^{9'}$ are the same or different from each other and each represent a hydrogen atom, a halogen atom, an alkyl group, a cyclic alkyl group, an aryl group, an alkenyl group, an alkoxy group, or an aryloxy group.

Examples of the thioxanthone compound represented by the above formula include thioxanthone, 2-isopropylthioxanthone, 4-isopropylthioxanthone, 2,3-diethylthioxanthone, 2,4-diethylthioxanthone, 2,4-dichlorothioxanthone, 2-methoxythioxanthone, 1-chloro-4-propoxythioxanthone, 2-cyclohexylthioxanthone, 4-cyclohexylthioxanthone, 2-vinylthioxanthone, 2,4-divinylthioxanthone, 2,4-diphenylthioxanthone, 2-butenyl-4-phenylthioxanthone, 2-methoxythioxanthone, and 2-p-octyloxyphenyl-4-ethylthioxanthone. Among these compounds, a compound in which one or two, particularly two of the $R^6$ to $R^9$ and $R^{6'}$ to $R^{9'}$ are substituted with alkyl groups is preferable, and 2,4-diethylthioxanthone is more preferable.

As for the method for adsorbing the photopolymerization initiator such as a benzophenone compound or a

thioxanthone compound onto the surface of the sliding portion, for example, in the case of using a benzophenone compound or a thioxanthone compound, the benzophenone compound or thioxanthone compound is dissolved in an organic solvent to prepare a solution; a surface portion of the sliding portion to be modified is treated with this solution, thereby adsorbing the compound onto the surface; and if necessary, the organic solvent is evaporated off by drying, whereby a polymerization initiation point is formed. The method for treating the surface is not particularly limited as long as the method allows the benzophenone or thioxanthone compound solution to be brought into contact with the surface of the sliding portion. For example, application or spraying of the benzophenone or thioxanthone compound solution, and immersion in the solution are suitable. If only part of the surface needs to be modified, it is sufficient to adsorb the photopolymerization initiator only onto such part of the surface. In this case, for example, application or spraying of the solution is suitable. As the solvent, methanol, ethanol, acetone, benzene, toluene, methyl ethyl ketone, ethyl acetate, THF, etc., can be used. Acetone is preferable because it does not swell the base gasket and it can be rapidly dried and evaporated off.

In the step 2, the monomer is radically polymerized starting from the polymerization initiation point formed in the step 1, to grow the polymer chain on the surface of the sliding portion.

The monomer may be appropriately selected from the above-described viewpoint. For example, acrylic acid, acrylic acid esters (methyl acrylate, ethyl acrylate, etc.), alkali metal salts of acrylic acid (sodium acrylate, potassium acrylate, etc.), amine salts of acrylic acid, acrylamide, dimethylacrylamide, diethylacrylamide, isopropylacrylamide, hydroxyethylacrylamide, acryloylmorpholine, methoxymethylacrylate, hydroxyethylacrylate, methacrylic acid, methacrylic acid esters (methyl methacrylate, ethyl methacrylate, etc.), alkali metal salts of methacrylic acid (sodium methacrylate, potassium methacrylate, etc.), amine salts of methacrylic acid, methacrylamide, dimethylmethacrylamide, diethylmethacrylamide, isopropylmethacrylamide, hydroxyethylmethacrylamide, methacryloylmorpholine, methoxymethyl methacrylate, hydroxyethyl methacrylate, acrylonitrile, etc., can be used as the monomer. These monomers may be used individually, or two or more thereof may be used in combination. Among these monomers, (meth)acrylic acid and (meth)acrylamide are preferable, and (meth)acrylic acid and (meth)acrylamide are more preferably used in combination.

As for the method for radically polymerizing the monomer in the step 2, a monomer-containing solution is applied (sprayed) to the surface of the sliding portion to which a benzophenone or thioxanthone compound or the like has been adsorbed, or the sliding portion is immersed in the monomer-containing solution, and the sliding portion is irradiated with UV light to allow radical polymerization (photoradical polymerization) to proceed in each case, whereby a polymer chain can be grown on the surface of the sliding portion. Furthermore, after the above application, the surface may be covered with glass, PET, polycarbonate, or the like that is transparent, and irradiated with light such as ultraviolet light to allow radical polymerization (photoradical polymerization) to proceed in each case, whereby a polymer chain can be grown on the surface of the sliding portion.

As the solvent for application (spraying), the method for application (spraying), the method for immersion, the conditions for irradiation, and the like, conventionally known materials and methods can be used. As the solution of the radically polymerizable monomer, an aqueous solution or a solution in an organic solvent that does not dissolve the photopolymerization initiator to be used (a benzophenone compound, a thioxanthone compound, etc.) is used. Furthermore, as the radically polymerizable monomer-containing liquid, a monomer-containing solution containing a known polymerization inhibitor such as 4-methylphenol can also be used.

After the application of the monomer-containing solution or after the immersion in the monomer or a solution of the monomer, radical polymerization of the monomer is allowed to proceed by light irradiation. Here, UV irradiation light sources with an emission wavelength mainly in the ultraviolet region, such as high-pressure mercury lamps, metal halide lamps, and LED lamps, can be suitably used. The irradiation amount of light may be selected appropriately in view of polymerization time and uniformity of the progress of the reaction. Moreover, in order to prevent inhibition of polymerization due to active gas such as oxygen in a reaction container or a reaction cylinder, oxygen is preferably removed from the reaction container or the reaction cylinder and the reaction solution during or before the light irradiation. Thus, for example, an inert gas, such as nitrogen gas or argon gas, is appropriately inserted into the reaction container, the reaction cylinder, and the reaction solution to discharge active gas such as oxygen from the reaction system and thereby replace the atmosphere in the reaction system with the inert gas. Furthermore, in order to prevent inhibition of the reaction due to oxygen or the like, for example, a measure is also appropriately taken in which a UV irradiation light source is disposed at a position where no air layer (oxygen content: 15% or higher) exists between the reaction container made of glass, plastics, or the like and the reaction solution or the base gasket.

The wavelength of the ultraviolet light is preferably 300 to 400 nm. Accordingly, a polymer chain can be favorably formed on the surface of the sliding portion. As the light source, a high-pressure mercury lamp, an LED with a center wavelength of 365 nm, an LED with a center wavelength of 375 nm, or the like, can be used. Irradiation with LED light having a wavelength of 355 to 380 nm is more preferable. In particular, an LED or the like that has a center wavelength of 365 nm, which is close to the excitation wavelength 366 nm of benzophenone, is preferable in view of efficiency. Light having a wavelength less than 300 nm may cleave molecules of the base gasket resulting in damage. Therefore, light having a wavelength of 300 nm or greater is preferable, and light having a wavelength of 355 nm or greater is further

preferable from the viewpoint of very small damage to the base gasket. Meanwhile, light having a wavelength greater than 400 nm is less likely to activate the photopolymerization initiator, so that it is difficult for the polymerization reaction to proceed. Therefore, light having a wavelength of 400 nm or less is preferable. Here, LED light is suitable because it has a narrow wavelength range and light having wavelengths other than the center wavelength is not emitted. Even with a mercury lamp or the like, the same effect as that with LED light can be achieved by cutting light having a wavelength less than 300 nm with a filter.

In the step 3, the silane compound is added to the surface of the polymer chain, and the fluoroalkyl group-containing silane compound and the perfluoroether group-containing silane compound is further reacted at a predetermined ratio to form (produce) a modified polymer chain. Accordingly, a modified polymer chain, in which the fluoroalkyl group-containing silane compound and the perfluoroether group-containing silane compound is added (reacted) at the predetermined ratio to the surface of the polymer chain, is formed, and desired performance is imparted.

The silane compound is not particularly limited, and a silane compound having no fluoroalkyl group, etc., can be used. Among these compounds, alkoxysilanes and modified alkoxysilanes are preferable, and alkoxysilanes are more preferable, because the effects of the present invention are more favorably achieved. The silane compounds may be used individually, or two or more thereof may be used in combination.

Examples of the alkoxysilanes include: monoalkoxysilanes such as trimethylmethoxysilane, triethylethoxysilane, tripropylpropoxysilane, and tributylbutoxysilane; dialkoxysilanes such as dimethyldimethoxysilane, diethyldiethoxysilane, dipropyldipropoxysilane, and dibutyldibutoxysilane; trialkoxysilanes such as methyltrimethoxysilane, ethyltriethoxysilane, propyltripropoxysilane, and butyltributoxysilane; and tetraalkoxysilanes such as tetramethoxysilane, tetraethoxysilane, tetrapropoxysilane, tetrabutoxysilane, dibutoxydiethoxysilane, butoxytriethoxysilane, and ethoxytriethoxysilane. These alkoxysilanes may be used individually, or two or more thereof may be used in combination. Among these alkoxysilanes, tetraalkoxysilanes are preferable, and tetramethoxysilane, tetraethoxysilane, tetrabutoxysilane, dibutoxydiethoxysilane, butoxytriethoxysilane, and ethoxytributoxysilane are more preferable, because the effects of the present invention are more favorably achieved.

The modified alkoxysilanes refer to alkoxysilanes having a substituent such as an amino group, a carboxyl group, a hydroxyl group, and an epoxy group, and each preferably have at least one substituent selected from the group consisting of an alkyl group, an amino group, a carboxyl group, a hydroxyl group, and an epoxy group.

The number of carbon atoms in each of the alkoxysilanes and modified alkoxysilanes is preferably 4 to 22 and more preferably 4 to 16 because the effects of the present invention are more favorably achieved.

The alkoxysilanes and modified alkoxysilanes each preferably have at least one group selected from the group consisting of a methoxy group, an ethoxy group, a propoxy group, and a butoxy group, more preferably have an ethoxy group and/or a butoxy group, and further preferably have an ethoxy group and a butoxy group, because the effects of the present invention are more favorably achieved.

As commercial products of the silane compound, Primer Coat PC-3B (manufactured by Fluoro Technology Co., Ltd., a butoxy/ethoxy tetraalkoxysilane represented by the following formula), etc., can be used.

[Chem. 7]

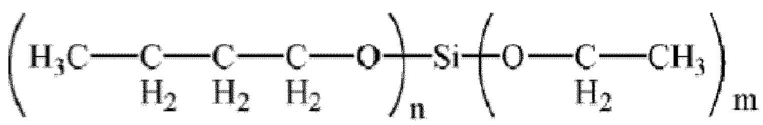

wherein m+n=4 and n>m>0 are satisfied as average values.

The method for adding the silane compound to the surface of the polymer chain in the step 3 is not particularly limited. For example, conventionally known methods, such as a method in which the object to be modified on which the polymer chain is formed is brought into contact with a solution of the silane compound, can be used as appropriate. The solution of the silane compound can be prepared by using a known solvent capable of dissolving the compound, such as water and alcohol, and adjusting the concentration as appropriate. The object to be modified can be brought into contact with the solution of the silane compound by any method that allows them to be brought into contact with each other, such as application, spraying, or immersion. The reaction temperature and the reaction time for the addition may be appropriately selected in accordance with the type of silane compound to be used, etc., and are, for example, 0.5 to 60 hours and 20 to 120°C.

In the step 3, after the silane compound is added to the surface of the polymer chain, the fluoroalkyl group-containing silane compound and the perfluoroether group-containing silane compound is then reacted at the predetermined ratio to form a modified polymer chain. Among these cases, both the fluoroalkyl group-containing silane compound and the perfluoroether group-containing silane compound are suitably reacted (added) to form a modified polymer chain, because the effects of the present invention are favorably achieved.

The fluoroalkyl group in the fluoroalkyl group-containing silane compound includes a group represented by the following formula.

[Chem. 8] $F_3C\text{-}(CF_2)_n\text{-}(CH_2)_m\text{-}$

wherein n is 0 to 5, and m is 0 to 8.

In the above formula, n is preferably 1 to 5 and more preferably 3 to 5, and m is preferably 1 to 6 and more preferably 2 to 6. Moreover, $0 \leq m+n \leq 10$ is preferably satisfied, and $0 \leq m+n \leq 7$ is more preferably satisfied.

Specific examples of the fluoroalkyl group include a 3,3,3-trifluoropropyl group, a 3,3,4,4,4-pentafluorobutyl group, a 3,3,4,4,5,5,6,6,6-nonafluorohexyl group, a 3,3,4,4,5,5,6,6,7,7,7-undecafluoroheptyl group, and a 3,3,4,4,5,5,6,6,7,7,8,8,8-tridecafluorooctyl group.

The fluoroalkyl group-containing silane compound is a compound represented by the following formula (1) and suitably used from the viewpoint of being able to economically impart various functions such as slidability, liquid leakage resistance, and protein adsorption inhibition performance.

[Chem. 9] $F_3C\text{-}(CF_2)_n\text{-}(CH_2)_m\text{-}Si(OR^1)_3$ (1)

wherein n is 0 to 5, m is 0 to 8, and $R^1$s each represent an alkyl group and may be the same or different from each other.

In formula (1), n is preferably 1 to 5 and more preferably 3 to 5, and m is preferably 1 to 6, and more preferably 2 to 6. Moreover, $0 \leq m+n \leq 10$ is preferably satisfied, and $0 \leq m+n \leq 7$ is more preferably satisfied. $R^1$s (alkyl groups) may each have a linear, branched, or cyclic structure, or two or more of these structures. The number of carbon atoms in each of $R^1$s is preferably 1 to 10, more preferably 1 to 5, and further preferably 1 to 3. Examples of the alkyl group for each $R^1$ include a methyl group, an ethyl group, and a propyl group.

Specific examples of the fluoroalkyl group-containing silane compound represented by the above formula (1) include 3,3,3-trifluoropropyltrimethoxysilane, 3,3,3-trifluoropropyltriethoxysilane, and 3,3,4,4,5,5,6,6,7,7,8,8,8-tridecafluorooctyltrimethoxysilane. These compounds can be used individually, or two or more thereof can be used in combination.

The perfluoroether group-containing silane compound is not particularly limited as long as it is a silane compound containing a perfluoroether group, and, for example, a compound represented by the following formula (2) or (3) can be suitably used.

[Chem. 10]

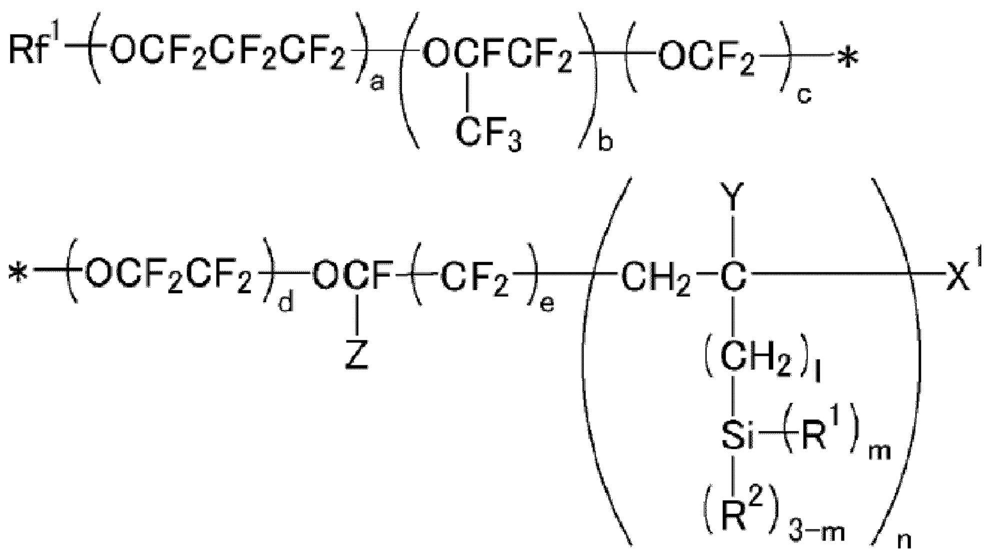

(2)

wherein $Rf^1$ represents a perfluoroalkyl group; Z represents fluorine or a trifluoromethyl group; a, b, c, d, and e are the same or different from each other and each represent an integer of 0 or 1 or more, a+b+c+d+e is 1 or more, the existing order of repeating units parenthesized by subscripts a, b, c, d, and e is not limited to that shown in the formula; Y represents hydrogen or a C1-C4 alkyl group; $X^1$ represents hydrogen, bromine, or iodine; $R^1$ represents a hydroxyl group or a hydrolyzable substituent such as a C1-C4 alkoxy group; $R^2$ represents hydrogen or a monovalent hydrocarbon group; 1 represents 0, 1, or 2; m represents 1, 2, or 3; and n represents an integer of 1 or more, and the two ends marked

by * are directly bonded to each other.

[Chem. 11]

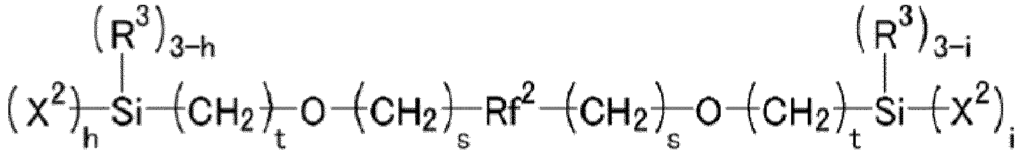

(3)

wherein $Rf^2$ represents a divalent group that contains a unit represented by $-(C_kF_{2k})O-$ (k is an integer from 1 to 6) and that has a non-branched linear perfluoropolyalkylene ether structure; $R^3$s are the same or different from each other and each represent a C1-C8 monovalent hydrocarbon group; $X^2$s are the same or different from each other and each represent a hydrolyzable group such as a C1-C4 alkoxy group or a halogen atom; each s is the same or different from each other and represents an integer from 0 to 2; each t is the same or different from each other and represents an integer from 1 to 5; and h and i are the same or different from each other and each represent 1, 2, or 3.

$Rf^1$ in formula (2) is not particularly limited as long as it is a perfluoroalkyl group that is contained in a common organic-containing fluoropolymer, and examples of $Rf^1$ in formula (2) include linear or branched C1-C16 groups. Among these groups, $CF_3$-, $C_2F_5$-, and $C_3F_7$- are preferable.

In formula (2), a, b, c, d, and e each represent the number of repeating units in the perfluoropolyether chain which forms the backbone of the fluorine-containing silane compound, and are each independently preferably 0 to 200 and more preferably 0 to 50. Moreover, a+b+c+d+e (sum of a to e) is preferably 1 to 100. Regarding the existing order of the repeating units parenthesized by subscripts a, b, c, d, and e in formula (2), these repeating units are shown in this order in formula (2), but the bonding order of these repeating units is not limited to this order and may be any order.

Examples of the C1-C4 alkyl group represented by Y in formula (2) include a methyl group, an ethyl group, a propyl group, and a butyl group, and the C1-C4 alkyl group may be linear or branched. When $X^1$ is bromine or iodine, the fluorine-containing silane compound easily forms a chemical bond.

The hydrolyzable substituent represented by $R^1$ in formula (2) is not particularly limited, and is preferably a halogen, $-OR^4$, $-OCOR^4$, $-OC(R^4)=C(R^5)_2$, $-ON=C(R^4)_2$, $-ON=CR^6$, or the like. Here, $R^4$ represents an aliphatic hydrocarbon group or an aromatic hydrocarbon group, $R^5$ represents hydrogen or a C1-C4 aliphatic hydrocarbon group, and $R^6$ represents a bivalent C3-C6 aliphatic hydrocarbon group. The hydrolyzable substituent represented by $R^1$ is more preferably chlorine, $-OCH_3$, or $-OC_2H_5$. The monovalent hydrocarbon group represented by $R^2$ is not particularly limited, and is preferably a methyl group, an ethyl group, a propyl group, a butyl group, or the like, and may be linear or branched.

In formula (2), 1 represents the number of carbon atoms in the alkylene group between the carbon in the perfluoropolyether chain and the silicon bonded thereto and is preferably 0; and m represents the number of substituents $R^1$ bonded to the silicon, and $R^2$ is bonded to a portion of the silicon to which $R^1$ is not bonded. The upper limit of n is not particularly limited, and is preferably an integer from 1 to 10.

Meanwhile, in formula (3), the group represented by $Rf^2$ is not particularly limited, and, when each s is 0, the ends of the $Rf^2$ group bonded to the oxygen atoms in formula (3) are preferably not oxygen atoms. Moreover, k in $Rf^2$ is preferably an integer from 1 to 4. Specific examples of the group represented by $Rf^2$ include $-CF_2CF_2O(CF_2CF_2CF_2O)_jCF_2CF_2-$ (wherein j is an integer of 1 or more, preferably 1 to 50, and more preferably 10 to 40) and $-CF_2(OC_2F_4)_p-(OCF_2)_q-$ (wherein p and q are each an integer of 1 or more, preferably 1 to 50, and more preferably 10 to 40, and the sum of p and q is an integer from 10 to 100, preferably 20 to 90, and more preferably 40 to 80, and the repeating units $(OC_2F_4)$ and $(OCF_2)$ are randomly arranged).

$R^3$ in formula (3) is preferably a C1-C8 monovalent hydrocarbon group, and examples of $R^3$ in formula (3) include: alkyl groups such as a methyl group, an ethyl group, a propyl group, a butyl group, a pentyl group, a hexyl group, a heptyl group, and an octyl group; cycloalkyl groups such as a cyclopentyl group and a cyclohexyl group; aryl groups such as a phenyl group, a tolyl group, and an xylyl group; aralkyl groups such as a benzyl group and a phenethyl group; and alkenyl groups such as a vinyl group, an aryl group, a butenyl group, a pentenyl group, and a hexenyl group. Among these groups, a methyl group is preferable.

Examples of the hydrolyzable group represented by $X^2$ in formula (3) include: alkoxy groups such as a methoxy group, an ethoxy group, a propoxy group, and a butoxy group; alkoxyalkoxy groups such as a methoxymethoxy group, a methoxyethoxy group, and an ethoxyethoxy group; alkenyloxy groups such as an allyloxy group and an isopropenoxy group; acyloxy groups such as an acetoxy group, a propionyloxy group, a butylcarbonyloxy group, and a benzoyloxy group; ketoxime groups such as a dimethylketoxime group, a methylethylketoxime group, a diethylketoxime group, a cyclopennoxime group, and a cyclohexanoxime group; amino groups such as an N-methylamino group, an N-ethylamino group, an N-propylamino group, an N-butylamino group, an N,N-dimethylamino group, an N,N-diethylamino group, and an N-cyclohexylamino group; amide groups such as an N-methylacetamide group, an N-ethylacetamide group, and an

N-methylbenzamide group; and aminooxy groups such as an N,N-dimethylaminooxy group and an N,N-diethylaminooxy group. Examples of the halogen atom represented by $X^2$ include a chlorine atom, a bromine atom, and an iodine atom. Among these groups, a methoxy group, an ethoxy group, an isopropenoxy group, and a chlorine atom are preferable.

In formula (3), s is preferably 1, and t is preferably 3. From the viewpoint of hydrolyzability, h and i are each preferably 3.

Examples of the perfluoroether group-containing silane compound also include a compound represented by the following formula (4).

[Chem. 12]

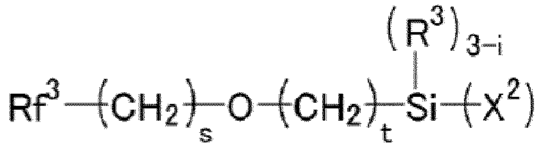

(4)

wherein $Rf^3$ represents a monovalent group that contains a unit represented by $-(C_kF_{2k})O-$ (k is an integer from 1 to 6) and that has a non-branched linear perfluoropolyalkylene ether structure; $R^3$s are the same or different from each other and each represent a C1-C8 monovalent hydrocarbon group; $X^2$s are the same or different from each other and each represent a hydrolyzable group such as a C1-C4 alkoxy group or a halogen atom; s represents an integer from 0 to 2; t represents an integer from 1 to 5; and each i is the same or different from each other and represents 1, 2, or 3.

In formula (4), the group represented by $Rf^3$ is not particularly limited, and, when s is 0, the end of the $Rf^3$ group bonded to the oxygen atom in formula (4) is preferably not an oxygen atom. Moreover, k in $Rf^3$ is preferably an integer from 1 to 4. Specific examples of the group represented by $Rf^3$ include $CF_2CF_2O(CF_2CF_2CF_2O)_jCF_2CF_2$- (wherein j is an integer of 1 or more, preferably 1 to 50, and more preferably 10 to 40) and $CF_3(OC_2F_4)_p$-$(OCF_2)_q$-(wherein p and q are each an integer of 1 or more, preferably 1 to 50, and more preferably 10 to 40, and the sum of p and q is an integer from 10 to 100, preferably 20 to 90, and more preferably 40 to 80, and the repeating units $(OC_2F_4)$ and $(OCF_2)$ are randomly arranged).

Examples of $R^3$ in formula (4) include groups that are the same as those for $R^3$ in formula (3). Examples of $X^2$ in formula (4) include groups that are the same as those for $X^2$ in formula (3). In formula (4), s is preferably 1, and t is preferably 3. In formula (4), each i is preferably 3 from the viewpoint of hydrolyzability.

The average molecular weight of the perfluoroether group-containing silane compound is preferably 1,000 to 10,000 in view of sustainability of mold release action. The average molecular weight can be measured by gel permeation chromatography (GPC) through conversion based on polystyrene standard.

Examples of commercial products of the perfluoroether group-containing silane compound include OPTOOL DSX (manufactured by Daikin Industries, Ltd.), KY-108, KY-164 (manufactured by Shin-Etsu Chemical Co., Ltd.), Fluorolink S10 (manufactured by Solvay Specialty Polymers Japan K.K.), Novec2702, Novec1720 (manufactured by 3M Japan Limited), FLUOROSURF series such as FLUOROSURF FG-5080SH (manufactured by Fluoro Technology Co., Ltd.), and SIP6720.72 (manufactured by Gelest, [Perfluoro(polypropyleneoxy)]methoxypropyltrimethoxysilane, $CF_3CF_2CF_2O(CF_2CF_2CF_2O)_nCH_2OCH_2CH_2CH_2Si(OCH_3)_3$)).

In the step 3, the method for reacting (adding) the fluoroalkyl group-containing silane compound and the perfluoroether group-containing silane compound at the predetermined ratio with the polymer chain having the silane compound added thereto is not particularly limited, and, for example, conventionally known methods, such as a method in which a solution containing the fluoroalkyl group-containing silane compound and the perfluoroether group-containing silane compound at a predetermined ratio is brought into contact with the object to be modified having the silane compound added to the surface of the polymer chain, can be used as appropriate. The solution containing the fluoroalkyl group-containing silane compound and/or the perfluoroether group-containing silane compound at the predetermined ratio can be prepared by using a known solvent capable of dissolving the compounds, such as water, perfluorohexane, acidic water, methanol, ethanol, and a mixed liquid of water and methanol or ethanol, and adjusting the concentration as appropriate. The solution can be brought into contact with the object to be modified, by any method that allows them to be brought into contact with each other, such as application, spraying, or immersion.

The reaction of the polymer chain having the silane compound added thereto and the fluoroalkyl group-containing silane compound and/or the perfluoroether group-containing silane compound at the predetermined ratio is preferably further maintained under the condition of a humidity of 50% or higher after the contact such as the above immersion. Accordingly, the reaction further proceeds, and the effects of the present invention are favorably achieved. The humidity is more preferably equal to or higher than 60% and further preferably equal to or higher than 80%. The upper limit of the humidity is not particularly limited, and is preferably, for example, equal to or lower than 100%. The retention time (reaction time) or the retention temperature (reaction temperature) may be appropriately set, and are

preferably, for example, 0.5 to 60 hours and 20 to 120°C.

In the step 3, the mixing ratio of the fluoroalkyl group-containing silane compound to the perfluoroether group-containing silane compound (fluoroalkyl group-containing silane compound : perfluoroether group-containing silane compound (mass ratio)) is 60:40 to 100:0, more preferably 60:40 to 90:10, and further preferably 65:35 to 85:15, from the viewpoint of imparting various functions such as slidability, liquid leakage resistance, and protein adsorption inhibition performance.

Moreover, in the syringe gasket, a polymer chain can also be formed on the surface of the sliding portion by a formation method including: a step I of radically polymerizing a monomer in the presence of a photopolymerization initiator to grow a polymer chain on the surface of the sliding portion; and a step II of adding a silane compound to the surface of the polymer chain and further reacting a fluoroalkyl group-containing silane compound and a perfluoroether group-containing silane compound at a predetermined ratio to form a modified polymer chain.

The step I can be carried out, for example, by using the above-described method of radical polymerization, such as bringing the photopolymerization initiator and the monomer into contact with the surface of the sliding portion and then irradiating the surface of the sliding portion with LED light of 300 to 400 nm, thereby forming a polymerization initiation point from the photopolymerization initiator and radically polymerizing the monomer starting from the polymerization initiation point to grow a polymer chain. As the photopolymerization initiator and the monomer used in the step I, a photopolymerization initiator and a monomer that are the same as described above can be used.

As for the method for radically polymerizing the monomer in the step I, a monomer-containing solution containing the photopolymerization initiator such as a benzophenone compound or a thioxanthone compound is applied (sprayed) to the surface of the sliding portion, or the sliding portion is immersed in the monomer-containing solution containing the photopolymerization initiator, and the sliding portion is irradiated with light such as ultraviolet light to allow radical polymerization (photoradical polymerization) to proceed, whereby a polymer chain can be grown on the surface of the sliding portion. Furthermore, for example, the above-described method in which the surface is covered with glass, PET, polycarbonate, or the like that is transparent, and the covered surface is irradiated with light such as ultraviolet light, can also be used. As the solvent for application (spraying), the method for application (spraying), the method for immersion, the conditions for irradiation, and the like, materials and methods that are the same as described above can be used.

As the methods for the reaction (addition or the like) of the silane compound and the reaction (addition or the like) of the fluoroalkyl group-containing silane compound and/or the perfluoroether group-containing silane compound in the step II, methods that are the same as in the step 3 described above can be used. As the silane compound, the fluoroalkyl group-containing silane compound, and the perfluoroether group-containing silane compound, compounds that are the same as described above can be used. A suitable mixing ratio of the fluoroalkyl group-containing silane compound to the perfluoroether group-containing silane compound is also the same as described above.

In the step 2 or the step I, two or more monomers may be simultaneously radically polymerized. Furthermore, a plurality of modified polymer chains may be grown on the surface of the sliding portion. In the present invention, modified polymer chains may be crosslinked with each other. In this case, ionic crosslinking, crosslinking with a hydrophilic group having an oxygen atom, and crosslinking with a halogen group such as iodine may be formed between the modified polymer chains.

By using the above-described polymer forming method, a syringe gasket having a modified sliding portion surface at least at a part thereof (a syringe gasket having a modified polymer chain fixed to at least a part of the surface of a sliding portion of a base gasket) can be produced. At least a part or the entirety of the surface of the sliding portion may be modified, and the entire surface of the base gasket may be modified.

The length of the modified polymer chain formed in the syringe gasket is preferably 200 to 7,000 nm and more preferably 300 to 2,000 nm. If the length is shorter than 200 nm, good slidability tends not to be achieved. If the length is longer than 7,000 nm, further improvement in slidability cannot be expected, and the cost of raw materials tends to increase due to the use of the expensive monomer. In addition, surface patterns formed by the surface treatment tend to be visible to the naked eyes and thus spoil the appearance or decrease the sealability.

In the syringe gasket (after the modified polymer chain is fixed), from the viewpoint of achieving both slidability and liquid leakage resistance, the first protrusion 17a on which the modified polymer chain is formed has a surface roughness Ra preferably equal to or less than 1.0, more preferably equal to or less than 0.8, and further preferably equal to or less than 0.6. The lower limit of the surface roughness Ra is not particularly limited, and the surface roughness Ra is preferably lower.

In the present specification, the surface roughness Ra is an arithmetic average height Ra defined by JIS B 0601-2001 and ISO 4287-1997.

From the viewpoint of achieving both slidability and liquid leakage resistance, the first protrusion 17a in the base gasket (before the modified polymer chain is fixed) has a surface roughness Ra preferably equal to or less than 1.0, more preferably equal to or less than 0.8, and further preferably equal to or less than 0.6. The lower limit of the surface roughness Ra is not particularly limited, and the surface roughness Ra is preferably lower.

In the syringe gasket (after the modified polymer chain is fixed), from the viewpoint of achieving both slidability

and liquid leakage resistance, the first protrusion 17a on which the modified polymer chain is formed has a surface roughness Rz preferably equal to or less than 25.0, more preferably equal to or less than 22.0, and further preferably equal to or less than 20.0. The lower limit of the surface roughness Rz is not particularly limited, and the surface roughness Rz is preferably lower.

In the present specification, the surface roughness Rz is a maximum height Rz defined by JIS B 0601-2001 and ISO 4287-1997.

In the syringe gasket (after the modified polymer chain is fixed), from the viewpoint of achieving both slidability and liquid leakage resistance, the first protrusion 17a on which the modified polymer chain is formed has a surface roughness Rv preferably equal to or less than 21.0, more preferably equal to or less than 18.0, and further preferably equal to or less than 16.5. The lower limit of the surface roughness Rv is not particularly limited, and the surface roughness Rv is preferably lower.

In the present specification, the surface roughness Rv is a maximum valley depth Rv defined by JIS B 0601-2001 and ISO 4287-1997.

The surface roughness Ra of the base gasket or the syringe gasket having the modified polymer chain fixed to the base gasket can be adjusted by a method of changing the surface roughness of a molding mold, etc. Specifically, the surface roughness Ra can be adjusted by changing the particle size of an abrasive to be used in a final polishing process during mold production. Examples of the abrasive include abrasive grains of diamond, alumina, silicon carbide, cubic crystal boron nitride, boron carbide, zirconium oxide, manganese oxide, and colloidal silica, and, for example, #46 to 100 defined by JIS R6001-1998, etc., can be suitably used.

As the material of the molding mold, known materials can be used, and examples of the material of the molding mold include carbon steel and precipitation-type stainless steel. The molding mold can be produced using a cutting method such as a method of performing grinding and polishing after cutting with a cemented carbide tool, a coated cemented carbide, a cBN sintered body, or the like.

The surface roughnesses Rz and Rv of the syringe gasket having the fixed modified polymer chain can be adjusted by a method of drying after reacting the fluoroalkyl group-containing silane compound and then washing with a solvent such as acetone. Specifically, if rapid drying is performed after only the modified polymer chain is washed, the surface tends to crack increasing Rz and Rv, and thus, when rapid drying is not performed but drying is performed at normal pressure and then gradual drying is performed by means of vacuum drying or the like, Rz and Rv are decreased, so that the above Rz and Rv can be achieved.

EXAMPLES

(Example 1)

An unvulcanized rubber sheet obtained by mixing triazine as a crosslinking agent into a chlorobutyl rubber (degree of unsaturation: 1 to 2%) containing isoprene units, and an inactive film having a thickness of 100 μm (PTFE film: one having a plasma-treated surface to be attached to a rubber sheet) were attached together, and molded with a vacuum press at 175°C for 10 minutes, while being vulcanized to be adhered to each other, to obtain a gasket (object to be modified) having the shape shown in FIG. 1. The obtained gasket was washed, dried, then immersed in a 1 wt% solution of benzophenone in acetone to adsorb the benzophenone onto the surface of the gasket, and dried.

The dried gasket was immersed in an aqueous solution of acrylic acid (1.25 M, 9.0 g of acrylic acid dissolved in 100 ml of water) in a glass reaction container, and was irradiated with LED-UV light having a wavelength of 365 nm for 25 minutes to cause radical polymerization to grow a polymer chain on the surface of the sliding portion. Thereafter, the surface was washed with water and dried.

Thereafter, the gasket was immersed in a 1 wt% solution of a silane compound (Primer Coat PC-3B (a butoxy/ethoxy tetraalkoxysilane represented by the above formula, manufactured by Fluoro Technology Co., Ltd.)) in butanol, and taken out from the solution. Then, the gasket was allowed to stand at 100°C under a humidity of 90% for 2 hours to cause reaction. The surface was washed with acetone, washed with water, and dried.

Then, the dried vulcanized rubber (gasket) was immersed in a 2% solution of a fluoroalkyl group-containing silane compound represented by the following formula (triethoxy-1H,1H,2H,2H-tridecafluoro-n-octylsilane: T1770 manufactured by Tokyo Chemical Industry Co., Ltd., fluoroalkyl group: $CF_3(CF_2)_5(CH_2)_2$-) in perfluorohexane, and taken out from the solution. Then, the gasket was allowed to stand at 70°C under a humidity of 90% for 8 hours to cause reaction. Thereafter, the gasket was washed with acetone and dried. Accordingly, a target syringe gasket was obtained.

[Chem. 13]

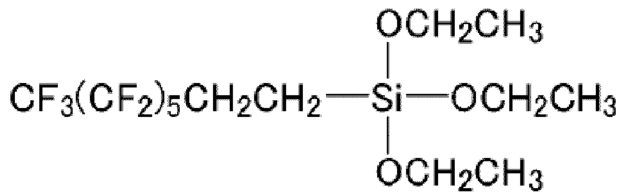

(Examples 2 to 4) (Examples 3 and 4 are not according to the invention)

Target syringe gaskets were obtained in the same manner as Example 1, except that the fluoroalkyl group-containing silane compound (triethoxy-1H,1H,2H,2H-tridecafluoro-n-octylsilane: T1770 manufactured by Tokyo Chemical Industry Co., Ltd., fluoroalkyl group: $CF_3(CF_2)_5(CH_2)_2$-) and a perfluoroether group-containing silane compound (OPTOOL DSX-E, manufactured by Daikin Industries, Ltd., the compound represented by the above formula (2)) were used at a mixing ratio described in Table 1, instead of the fluoroalkyl group-containing silane compound (triethoxy-1H,1H,2H,2H-tridecafluoro-n-octylsilane: T1770 manufactured by Tokyo Chemical Industry Co., Ltd., fluoroalkyl group: $CF_3(CF_2)_5(CH_2)_2$-).

(Example 5)

An unvulcanized rubber sheet obtained by mixing triazine as a crosslinking agent into a chlorobutyl rubber (degree of unsaturation: 1 to 2%) containing isoprene units, and an inactive film having a thickness of 100 $\mu$m (PTFE film: one having a plasma-treated surface to be attached to a rubber sheet) were attached together, and molded with a vacuum press at 175°C for 10 minutes, while being vulcanized to be adhered to each other, to obtain a gasket (object to be modified) having the shape shown in FIG. 1. The obtained gasket was washed, dried, then immersed in a 1 wt% solution of benzophenone in acetone to adsorb the benzophenone onto the surface of the gasket, and dried.

The dried base gasket was immersed in a mixed aqueous solution containing acrylic acid and acrylamide at a ratio of 25:75 (2.5 M, 2.255 g of acrylic acid and 6.7 g of acrylamide dissolved in 100 mL of water) in a glass reaction container, and was irradiated with LED-UV light having a wavelength of 365 nm for 45 minutes to cause radical polymerization to grow a polymer chain on the surface of the sliding portion. Thereafter, the surface was washed with water and dried.

Thereafter, the gasket was immersed in a 1 wt% solution of a silane compound (Primer Coat PC-3B (a butoxy/ethoxy tetraalkoxysilane represented by the above formula, manufactured by Fluoro Technology Co., Ltd.)) in butanol, and taken out from the solution. Then, the gasket was allowed to stand at 100°C under a humidity of 90% for 2 hours to cause reaction. The surface was washed with acetone, washed with water, and dried.

Then, the dried vulcanized rubber (gasket) was immersed in a 2% solution of a fluoroalkyl group-containing silane compound represented by the following formula (triethoxy-1H,1H,2H,2H-tridecafluoro-n-octylsilane: T1770 manufactured by Tokyo Chemical Industry Co., Ltd., fluoroalkyl group: $CF_3(CF_2)_5(CH_2)_2$-) in perfluorohexane, and taken out from the solution. Then, the gasket was allowed to stand at 70°C under a humidity of 90% for 8 hours to cause reaction. Thereafter, the gasket was washed with acetone and dried. Accordingly, a target syringe gasket was obtained.

[Chem. 14]

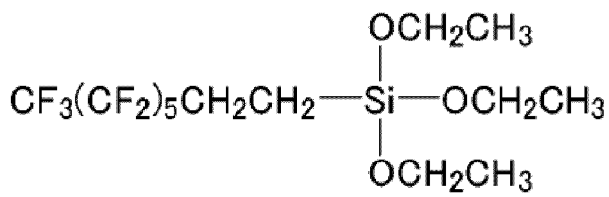

(Examples 6 to 8) (Examples 7 and 8 are not according to the invention)

Target syringe gaskets were obtained in the same manner as Example 5, except that the fluoroalkyl group-containing silane compound (triethoxy-1H,1H,2H,2H-tridecafluoro-n-octylsilane: T1770 manufactured by Tokyo Chemical Industry Co., Ltd., fluoroalkyl group: $CF_3(CF_2)_5(CH_2)_2$-) and a perfluoroether group-containing silane compound (OPTOOL DSX-E, manufactured by Daikin Industries, Ltd., the compound represented by the above formula (2)) were

used at a mixing ratio described in Table 1, instead of the fluoroalkyl group-containing silane compound (triethoxy-1H,1H,2H,2H-tridecafluoro-n-octylsilane: T1770 manufactured by Tokyo Chemical Industry Co., Ltd., fluoroalkyl group: $CF_3(CF_2)_5(CH_2)_2$-).

(Examples 9 and 10)

Target syringe gaskets were obtained in the same manner as Example 6, except that the roughness of the rubber surface (base gasket surface) described in Table 1 was changed. The roughness of the rubber surface was set by adjusting the surface roughness of a mold.

(Example 11)

A target syringe gasket was obtained in the same manner as Example 6, except that the fluoroalkyl group-containing silane compound was changed to $CF_3(CF_2)_3CH_2CH_2Si(OCH_2CH_3)_3$.

(Comparative Example 1)

A vulcanized rubber gasket (vulcanized at 175°C for 10 minutes) obtained by crosslinking a chlorobutyl rubber (degree of unsaturation: 1 to 2%) containing isoprene units with use of triazine, was used.

(Comparative Example 2)

An unvulcanized rubber sheet obtained by mixing triazine as a crosslinking agent into a chlorobutyl rubber (degree of unsaturation: 1 to 2%) containing isoprene units, and an inactive film having a thickness of 100 μm (PTFE film: one having a plasma-treated surface to be attached to a rubber sheet) were attached together, and molded with a vacuum press at 175°C for 10 minutes, while being vulcanized to be adhered to each other, to obtain a syringe gasket having the shape shown in FIG. 1.

The syringe gaskets and the like produced in the Examples and the Comparative Examples were evaluated by the following methods.

[Surface Roughnesses Ra, Rz, Rv]

According to JIS B 0601-2001 (ISO 4287-1997), Ra (arithmetic average height), Rz (maximum height), and Rv (maximum valley depth) of each vulcanized rubber (base gasket) and each surface-modified elastic body (after the modified polymer chain was fixed) were measured.

[Length of Modified Polymer Chain (Entire Polymer Chain)]

The length of the modified polymer chain formed on the vulcanized rubber surface was measured on a cross-section of the modified rubber on which the modified polymer chain was formed, using a SEM, at an acceleration voltage of 15 kV and at a magnification of 1,000 times. The thickness of a photographed polymer layer was regarded as the length of the modified polymer chain.

[Slidability (Friction Resistance)]

To measure the friction resistance of the surface, each of the syringe gaskets produced in the Examples and the Comparative Examples was set in a COP resin syringe of an injector, and pushed using a tensile tester, and the friction resistance was measured at that time (push rate: 30 mm/min). The friction resistance of the Examples and the Comparative Examples is indicated as a friction resistance index using the following equation, with a friction resistance index of Comparative Example 1 being set to 100. A lower index indicates that the friction resistance is lower and the slidability is better.

$$\text{(Friction resistance index)} = \text{(friction resistance of each example)}/\text{(friction resistance of Comparative Example 1)} \times 100$$

[Liquid Leakage Resistance]

Each of the syringe gaskets produced in the Examples and the Comparative Examples was set in a COP resin syringe of an injector, an aqueous solution of red food coloring was put in the syringe, and the syringe was capped and kept at 40°C for 2 weeks. Thereafter, the state of liquid leakage was visually checked and evaluated by the following four criteria.

A: There was no red (pink) stain of red food coloring on the first protrusion closest to the top surface.
B: Red (pink) stains of red food coloring were slightly observed on the upper half of the first protrusion closest to the top surface.
C: Red (pink) stains of red food coloring were observed, on the first protrusion closest to the top surface, to the lower portion of the protrusion.
D: Red (pink) stains were observed beyond the first protrusion closest to the top surface.

[Protein Adsorption Amount]

The surface of each of the obtained samples (syringe gaskets) was brought into contact with a 1 mg/ml bovine serum albumin (BSA) solution and allowed to stand at 37°C for 3 hours. The sample surface was gently washed with phosphate buffered saline to obtain a protein adsorption sample. The entire protein adsorption sample was put in a 50-ml centrifuge tube, and the protein adsorbed on the sample surface was extracted according to the method described in 3.6 Water-soluble protein in JIS T9010: 1999 "Test methods relevant to biological safety of rubber products". 0.5 ml of a 0.1 mol/l sodium hydroxide aqueous solution was accurately added to the obtained protein, and the obtained protein was dissolved in the aqueous solution to obtain a sample solution. Moreover, a solution obtained by performing the same operations without putting any sample was used as an operation blank.

0.2 ml of the sample solution and 0.2 ml of each of standard solutions (BSA solutions (5 to 100 μg/ml)) were accurately taken, and the amount of protein was quantified by the Lowry method. A calibration curve was created from the BSA concentrations (μg/ml) and absorbance of the standard solutions, the protein concentration (μg/ml) per 1 ml of the sample solution was calculated by using the calibration curve, and the value was converted to a value per area of the syringe gasket.

[

Table 1]

| | Comparative Example 1 | Example 1 | Example 2 | Example 3 | Example 4 | Example 5 | Example 6 | Example 7 | Example 8 | Example 9 | Example 10 | Comparative Example 2 | Example 11 |
|---|---|---|---|---|---|---|---|---|---|---|---|---|---|
| Presence/absence of inactive film | Absence | Prese nce | Presence | Presence | Presence | Presence | Prese nce | Presence | Presence | Presence | Presence | Prese nce | Presence |
| Acrylic acid/acrylamide | - | 100/0 | 100/0 | 100/0 | 100/0 | 25/75 | 25/75 | 25/75 | 25/75 | 25/75 | 25/75 | - | 25/75 |
| Silane compound (PC-3B) | | Prese nce | Presence | Presence | Presence | Presence | Prese nce | Presence | Presence | Presence | Presence | - | Presence |
| Fluoroalkyl group-containing silane compound (T1770) / perfluoroether group-containing silane compound (DSX-E) | - | 100/0 | 75/25 | 25/75 | 0/100 | 100/0 | 75/25 | 25/75 | 0/100 | 75/25 | 75/25 | - | - |
| Fluoroalkyl group-containing silane compound ($CF_3(CF_2)_3CH_2CH_2Si(OCH_2CH_3)_3$) / perfluoroether group-containing silane compound (DSX-E) | - | - | - | - | - | - | - | - | - | - | - | - | 75/25 |
| Surface roughness Ra (base gasket) | 0.45 | 0.45 | 0.45 | 0.45 | 0.45 | 0.45 | 0.45 | 0.45 | 0.45 | 0.6 | 0.3 | 0.45 | 0.45 |
| Surface roughness Ra (after fixing modified polymer chain) | - | 0.57 | 0.55 | 0.55 | 0.5 | 0.55 | 0.53 | 0.51 | 0.49 | 0.7 | 0.39 | 0.45 | 0.54 |

(continued)

| | Comparative Example 1 | Example 1 | Example 2 | Example 3 | Example 4 | Example 5 | Example 6 | Example 7 | Example 8 | Example 9 | Example 10 | Comparative Example 2 | Example 11 |
|---|---|---|---|---|---|---|---|---|---|---|---|---|---|
| Slidability | 100 | 2.85 | 2.3 | 1.9 | 1.85 | 2.45 | 1.95 | 1.25 | 1.1 | 2.15 | 1.8 | 28.5 | 2.1 |
| Liquid leakage resistance | C | A | A | A | A | A | A | A | A | B | A | C | A |
| Protein adsorption amount ($\mu g/cm^2$) | 1.62 | 0.34 | 0.33 | 0.33 | 0.33 | 0.33 | 0.33 | 0.33 | 0.33 | 0.33 | 0.33 | 1.58 | 0.35 |
| Length of modified polymer chain (nm) | 0 | 160 | 165 | 165 | 180 | 240 | 250 | 255 | 270 | 260 | 240 | - | 235 |

[Table 2]

| | Example 6 |
|---|---|
| Presence/absence of inactive film | Presence |
| Acrylic acid/acrylamide | 25/75 |
| Silane compound (PC-3B) | Presence |
| Fluoroalkyl group-containing silane compound (T1770) / perfluoroether group-containing silane compound (DSX-E) | 75/25 |
| Fluoroalkyl group-containing silane compound ($CF_3(CF_2)_3CH_2CH_2Si(OCH_2CH_3)_3$) / perfluoroether group-containing silane compound (DSX-E) | - |
| Surface roughness Rz (after fixing modified polymer chain) | 22.3 |
| Surface roughness Rv (after fixing modified polymer chain) | 16.6 |
| Slidability | 1.95 |
| Liquid leakage resistance | A |
| Protein adsorption amount ($\mu g/cm^2$) | 0.33 |
| Length of modified polymer chain (nm) | 250 |

From Tables 1 and 2, the surface of each of the syringe gaskets of the Examples had greatly decreased friction resistance, and the slidability was good. Moreover, good liquid leakage resistance and protein adsorption inhibition performance were also achieved. In particular, when the silane compound containing a large amount of the fluoroalkyl group-containing silane compound was reacted (added) on the surface, very good liquid leakage resistance was achieved. Regarding the protein adsorption inhibition performance, it was found that a protein adsorption amount larger than 1.0 $\mu g/cm^2$ indicates that protein was in a multilayered state, and it is considered that adsorption was dominant, but a protein adsorption amount equal to or less than 0.7 $\mu g/cm^2$ indicates that protein was roughly in a single-layered state, adsorption and desorption occurred at the same time, and it was in a good state where the adsorption was not dominant (the adsorption did not increase any more).

Therefore, in the case of use as the gasket of the plunger of an injector, the friction of the plunger against the syringe is reduced which sufficient liquid leakage resistance is achieved, so that medical treatment with the injector can be easily and accurately performed. In addition, protein adsorption can also be sufficiently inhibited.

Furthermore, the above-described effects can also be expected when modified polymer chains are formed on the surfaces of the grooves formed on the tread or of the sidewalls of tires for use on vehicles such as passenger cars, on the surfaces of diaphragms, on the sliding surfaces of skis or snowboards, or on the surfaces of swimsuits, road signs, sign boards, etc.

## Claims

**1.** A syringe gasket (1) comprising a liquid contact portion (12) and a sliding portion (13), wherein

an inactive film (15) is layered on the liquid contact portion (12),
no inactive film (15) is layered on the sliding portion (13),
a modified polymer chain is formed on a surface of the sliding portion (13),
the modified polymer chain is obtained by adding a silane compound to a surface of a polymer chain and further reacting a fluoroalkyl group-containing silane compound and a perfluoroether group-containing silane compound or further reacting a fluoroalkyl group-containing silane compound,
a mass ratio of the fluoroalkyl group-containing silane compound to the perfluoroether group-containing silane compound is 60:40 to 100:0, and
the fluoroalkyl group-containing silane compound is a compound represented by the following formula (1),

$$F_3C\text{-}(CF_2)_n\text{-}(CH_2)_m\text{-}Si(OR^1)_3 \qquad (1)$$

wherein n is 0 to 5, m is 0 to 8, and $R^1$s each represent an alkyl group and may be the same or different from each other.

**2.** The syringe gasket (1) according to claim 1, wherein the inactive film (15) is formed from an olefin-based resin and/or at least one fluorine resin selected from the group consisting of a tetrafluoroethylene polymer, a tetrafluoroethylene-ethylene copolymer, a modified tetrafluoroethylene polymer, a modified tetrafluoroethylene-ethylene copolymer, and a chlorofluoroethylene polymer.

**3.** The syringe gasket (1) according to claim 1 or 2, wherein the modified polymer chain is formed by a formation method including: a step 1 of forming a polymerization initiation point on the surface of the sliding portion (13); a step 2 of radically polymerizing a monomer starting from the polymerization initiation point to grow a polymer chain on the surface of the sliding portion (13); and a step 3 of adding the silane compound to a surface of the polymer chain and further reacting the fluoroalkyl group-containing silane compound and/or the perfluoroether group-containing silane compound to form the modified polymer chain.

**4.** The syringe gasket (1) according to claim 1 or 2, wherein the modified polymer chain is formed by a formation method including: a step I of radically polymerizing a monomer in the presence of a photopolymerization initiator to grow a polymer chain on the surface of the sliding portion (13); and a step II of adding the silane compound to a surface of the polymer chain and further reacting the fluoroalkyl group-containing silane compound and/or the perfluoroether group-containing silane compound to form the modified polymer chain.

**5.** The syringe gasket (1) according to claim 3 or 4, wherein the monomer is at least one monomer selected from the group consisting of acrylic acid, acrylic acid esters, alkali metal salts of acrylic acid, amine salts of acrylic acid, acrylamide, dimethylacrylamide, diethylacrylamide, isopropylacrylamide, hydroxyethylacrylamide, acryloylmorpholine, methoxymethylacrylate, hydroxyethylacrylate, methacrylic acid, methacrylic acid esters, alkali metal salts of methacrylic acid, amine salts of methacrylic acid, methacrylamide, dimethylmethacrylamide, diethylmethacrylamide, isopropylmethacrylamide, hydroxyethylmethacrylamide, methacryloylmorpholine, methoxymethyl methacrylate, hydroxyethyl methacrylate, and acrylonitrile.

**6.** The syringe gasket (1) according to any one of claims 1 to 5, wherein the perfluoroether group-containing silane compound is a compound represented by the following formula (2) or (3),

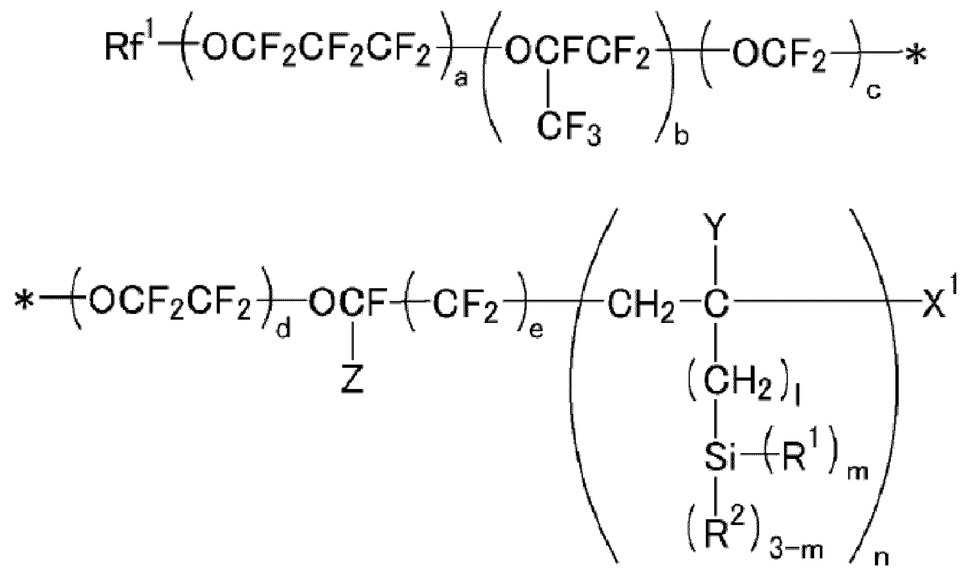

(2)

wherein $Rf^1$ represents a perfluoroalkyl group; Z represents fluorine or a trifluoromethyl group; a, b, c, d, and e are the same or different from each other and each represent an integer of 0 or 1 or more, a+b+c+d+e is 1 or more, the existing order of repeating units parenthesized by subscripts a, b, c, d, and e is not limited to that shown in the formula; Y represents hydrogen or a C1-C4 alkyl group; $X^1$ represents hydrogen, bromine, or iodine; $R^1$ represents a hydroxyl group or a hydrolyzable substituent; $R^2$ represents hydrogen or a monovalent hydrocarbon group; 1 represents 0, 1, or 2; m represents 1, 2, or 3; and n represents an integer of 1 or more, and the two ends marked

by * are directly bonded to each other,

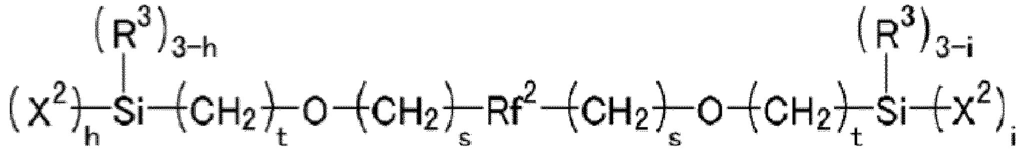

(3)

wherein $Rf^2$ represents a divalent group that contains a unit represented by $-(C_kF_{2k})O-$ (k is an integer from 1 to 6) and that has a non-branched linear perfluoropolyalkylene ether structure; $R^3$s are the same or different from each other and each represent a C1-C8 monovalent hydrocarbon group; $X^2$s are the same or different from each other and each represent a hydrolyzable group or a halogen atom; each s is the same or different from each other and represents an integer from 0 to 2; each t is the same or different from each other and represents an integer from 1 to 5; and h and i are the same or different from each other and each represent 1, 2, or 3.

**7.** The syringe gasket (1) according to any one of claims 1 to 6, wherein the modified polymer chain has a length of 200 to 7,000 nm, as measured according to the description.

**8.** The syringe gasket (1) according to any one of claims 1 to 7, wherein

the modified polymer chain is fixed to at least a part of a surface of a base gasket (11),
the sliding portion (13) has a plurality of annular protrusions (17a, 17b, 17c) on a sliding surface thereof, and the base gasket (11) has a surface roughness Ra, as measured according to the description, equal to or less than 1.0 at a first protrusion (17a) closest to a top surface among the annular protrusions (17a, 17b, 17c).

**9.** The syringe gasket (1) according to claim 8, wherein the surface roughness Ra of the base gasket (11) is equal to or less than 0.8.

**10.** The syringe gasket (1) according to claim 8, wherein the surface roughness Ra of the base gasket (11) is equal to or less than 0.6.

**11.** The syringe gasket (1) according to any one of claims 1 to 10, wherein

the sliding portion (13) has a plurality of annular protrusions (17a, 17b, 17c) on a sliding surface thereof, and a first protrusion (17a) closest to a top surface among the annular protrusions (17a, 17b, 17c) has a surface roughness Ra, as measured according to the description , equal to or less than 1.0.

**12.** The syringe gasket (1) according to claim 11, wherein the surface roughness Ra is equal to or less than 0.8.

**13.** The syringe gasket (1) according to claim 11, wherein the surface roughness Ra is equal to or less than 0.6.

**14.** The syringe gasket (1) according to any one of claims 1 to 13, wherein

the sliding portion (13) has a plurality of annular protrusions (17a, 17b, 17c) on a sliding surface thereof, and a first protrusion (17a) closest to a top surface among the annular protrusions (17a, 17b, 17c) has a surface roughness Rz, as measured according to the description, equal to or less than 25.0.

**15.** The syringe gasket (1) according to any one of claims 1 to 14, wherein

the sliding portion (13) has a plurality of annular protrusions (17a, 17b, 17c) on a sliding surface thereof, and a first protrusion (17a) closest to a top surface among the annular protrusions (17a, 17b, 17c) has a surface roughness Rv, as measured according to the description, equal to or less than 21.0.

## Patentansprüche

**1.** Spritzendichtung (1), umfassend einen Flüssigkeitskontaktabschnitt (12) und einen Gleitabschnitt (13), wobei

ein inaktiver Film (15) auf den Flüssigkeitskontaktabschnitt (12) aufgeschichtet ist,
kein inaktiver Film (15) auf den Gleitabschnitt (13) aufgeschichtet ist,
eine modifizierte Polymerkette auf einer Oberfläche des Gleitabschnitts (13) gebildet ist,
die modifizierte Polymerkette erhalten ist durch Hinzufügen einer Silanverbindung zu einer Oberfläche einer Polymerkette und zusätzliches Umsetzen mit einer Fluoralkylgruppen-haltigen Silanverbindung und einer Perfluorethergruppen-haltigen Silanverbindung oder zusätzliches Umsetzen mit einer Fluoralkylgruppen-haltigen Silanverbindung,
ein Massenverhältnis der Fluoralkylgruppen-haltigen Silanverbindung zu der Perfluorethergruppen-haltigen Silanverbindung 60:40 bis 100:0 beträgt, und
die Fluoralkylgruppen-haltige Silanverbindung eine durch die folgende Formel (1) dargestellte Verbindung ist,

$$F_3C\text{-}(CF_2)_n\text{-}(CH_2)_m\text{-}Si(OR^1)_3 \qquad (1)$$

wobei n 0 bis 5 ist, m 0 bis 8 ist, und $R^1$ jeweils eine Alkylgruppe darstellen und gleich oder voneinander verschieden sein können.

**2.** Spritzendichtung (1) nach Anspruch 1, wobei der inaktive Film (15) mit einem Olefin-basierten Harz und/oder mindestens einem Fluorharz gebildet ist, welches ausgewählt ist aus der Gruppe bestehend aus einem Tetrafluorethylen-Polymer, einem Tetrafluorethylen-Ethylen-Copolymer, einem modifizierten Tetrafluorethylen-Polymer, einem modifizierten Tetrafluorethylen-Ethylen-Copolymer und einem Chlorfluorethylen-Polymer.

**3.** Spritzendichtung (1) nach Anspruch 1 oder 2, wobei die modifizierte Polymerkette durch ein Bildungsverfahren gebildet ist, welches umfasst:
einen Schritt 1 des Bildens eines Polymerisations-Initiations-Punktes auf der Oberfläche des Gleitabschnitts (13);
einen Schritt 2 des radikalischen Polymerisierens eines Monomers, ausgehend von dem Polymerisations-Initiations-Punkt, um eine Polymerkette auf der Oberfläche des Gleitabschnitts (13) zu erzeugen; und einen Schritt 3 des Hinzufügens der Silanverbindung zu einer Oberfläche der Polymerkette und zusätzlichen Umsetzens mit der Fluoralkylgruppen-haltigen Silanverbindung und/oder der Perfluorethergruppen-haltigen Silanverbindung, um die modifizierte Polymerkette zu bilden.

**4.** Spritzendichtung (1) nach Anspruch 1 oder 2, wobei die modifizierte Polymerkette durch ein Bildungsverfahren gebildet ist, welches umfasst:
einen Schritt I des radikalischen Polymerisierens eines Monomers in der Gegenwart eines Photopolymerisationsinitiators, um eine Polymerkette auf der Oberfläche des Gleitabschnitts (13) zu erzeugen; und einen Schritt II des Hinzufügens der Silanverbindung zu einer Oberfläche der Polymerkette und zusätzlichen Umsetzens mit der Fluoralkylgruppen-haltigen Silanverbindung und/oder der Perfluorethergruppen-haltigen Silanverbindung, um die modifizierte Polymerkette zu bilden.

**5.** Spritzendichtung (1) nach Anspruch 3 oder 4, wobei das Monomer mindestens ein Monomer ist, welches ausgewählt ist aus der Gruppe bestehend aus Acrylsäure, Acrylsäureestern, Alkalimetallsalzen der Acrylsäure, Aminsalzen der Acrylsäure, Acrylamid, Dimethylacrylamid, Diethylacrylamid, Isopropylacrylamid, Hydroxyethylacrylamid, Acryloylmorpholin, Methoxymethylacrylat, Hydroxyethylacrylat, Methacrylsäure, Methacrylsäureestern, Alkalimetallsalzen der Methacrylsäure, Aminsalzen der Methacrylsäure, Methacrylamid, Dimethylmethacrylamid, Diethylmethacrylamid, Isopropylmethacrylamid, Hydroxyethylmethacrylamid, Methacryloylmorpholin, Methoxymethylmethacrylat, Hydroxyethylmethacrylat und Acrylnitril.

**6.** Spritzendichtung (1) nach einem der Ansprüche 1 bis 5, wobei die Perfluorethergruppen-haltige Silanverbindung eine durch die folgende Formel (2) oder (3) dargestellte Verbindung ist,

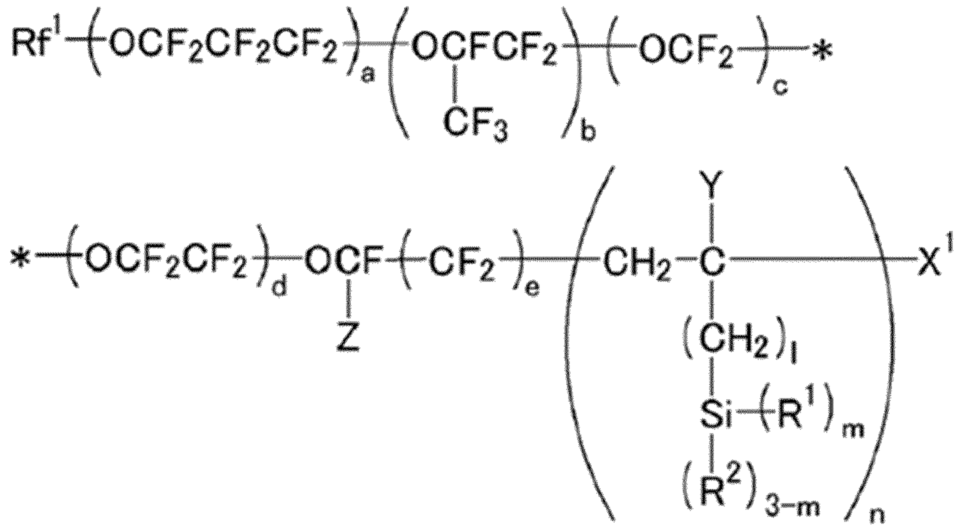

(2)

wobei $Rf^1$ eine Perfluoralkylgruppe darstellt; Z Fluor oder eine Trifluormethylgruppe darstellt; a, b, c, d und e gleich oder voneinander verschieden sind und jeweils eine ganze Zahl von 0 oder 1 oder mehr darstellen, a+b+c+d+e 1 oder mehr ist, die tatsächliche Reihenfolge mit Indizes a, b, c, d und e in Klammern gesetzter Wiederholungseinheiten nicht auf diejenige beschränkt ist, die in der Formel gezeigt ist; Y Wasserstoff oder eine C1-C4-Alkylgruppe darstellt; $X^1$ Wasserstoff, Brom oder Iod darstellt; $R^1$ eine Hydroxygruppe oder einen hydrolysierbaren Substituenten darstellt; $R^2$ Wasserstoff oder eine einwertige Kohlenwasserstoffgruppe darstellt; l 0, 1 oder 2 darstellt; m 1, 2 oder 3 darstellt; und n eine ganze Zahl von 1 oder mehr darstellt, und die beiden durch * gekennzeichneten Enden direkt miteinander verbunden sind,

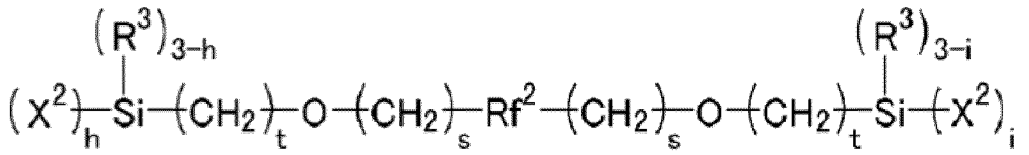

(3)

wobei $Rf^2$ eine zweiwertige Gruppe darstellt, die eine durch $-(C_kF_{2k})O-$ dargestellte Einheit enthält (k ist eine ganze Zahl von 1 bis 6) und die eine nicht-verzweigte, lineare Perfluorpolyalkylenether-Struktur aufweist; $R^3$ gleich oder voneinander verschieden sind und jeweils eine einwertige C1-C8-Kohlenwasserstoffgruppe darstellen; $X^2$ gleich oder voneinander verschieden sind und jeweils eine hydrolysierbare Gruppe oder ein Halogenatom darstellen; s jeweils gleich oder voneinander verschieden ist und eine ganze Zahl von 0 bis 2 darstellt; t jeweils gleich oder verschieden ist und eine ganze Zahl von 1 bis 5 darstellt; und h und i gleich oder voneinander verschieden sind und jeweils 1, 2 oder 3 darstellen.

**7.** Spritzendichtung (1) nach einem der Ansprüche 1 bis 6, wobei die modifizierte Polymerkette eine Länge, gemessen gemäß der Beschreibung, von 200 bis 7000 nm aufweist.

**8.** Spritzendichtung (1) nach einem der Ansprüche 1 bis 7, wobei

die modifizierte Polymerkette auf zumindest einen Teil einer Oberfläche einer Basisdichtung (11) aufgebracht ist,
der Gleitabschnitt (13) eine Mehrzahl ringförmiger Vorsprünge (17a, 17b, 17c) auf seiner Gleitoberfläche aufweist, und
die Basisdichtung (11) eine Oberflächenrauigkeit Ra, gemessen gemäß der Beschreibung, von gleich oder weniger als 1,0 an einem ersten Vorsprung (17a) aufweist, der von den ringförmigen Vorsprüngen (17a, 17b, 17c) einer oberen Oberfläche am nächsten liegt.

**9.** Spritzendichtung (1) nach Anspruch 8, wobei die Oberflächenrauigkeit Ra der Basisdichtung (11) gleich oder weniger als 0,8 ist.

**10.** Spritzendichtung (1) nach Anspruch 8, wobei die Oberflächenrauigkeit Ra der Basisdichtung (11) gleich oder weniger als 0,6 ist.

**11.** Spritzendichtung (1) nach einem der Ansprüche 1 bis 10, wobei

der Gleitabschnitt (13) eine Mehrzahl ringförmiger Vorsprünge (17a, 17b, 17c) auf seiner Gleitoberfläche aufweist, und
ein erster Vorsprung (17a), der von den ringförmigen Vorsprüngen (17a, 17b, 17c) einer oberen Oberfläche am nächsten liegt, eine Oberflächenrauigkeit Ra, gemessen gemäß der Beschreibung, von gleich oder weniger als 1,0 aufweist.

**12.** Spritzendichtung (1) nach Anspruch 11, wobei die Oberflächenrauigkeit Ra gleich oder weniger als 0,8 ist.

**13.** Spritzendichtung (1) nach Anspruch 11, wobei die Oberflächenrauigkeit Ra gleich oder weniger als 0,6 ist.

**14.** Spritzendichtung (1) nach einem der Ansprüche 1 bis 13, wobei

der Gleitabschnitt (13) eine Mehrzahl ringförmiger Vorsprünge (17a, 17b, 17c) auf seiner Gleitoberfläche aufweist, und
ein erster Vorsprung (17a), der von den ringförmigen Vorsprüngen (17a, 17b, 17c) einer oberen Oberfläche am nächsten liegt, eine Oberflächenrauigkeit Rz, gemessen gemäß der Beschreibung, von gleich oder weniger als 25,0 aufweist.

**15.** Spritzendichtung (1) nach einem der Ansprüche 1 bis 14, wobei

der Gleitabschnitt (13) eine Mehrzahl ringförmiger Vorsprünge (17a, 17b, 17c) auf seiner Gleitoberfläche aufweist, und
ein erster Vorsprung (17a), der von den ringförmigen Vorsprüngen einer oberen Oberfläche am nächsten liegt, eine Oberflächenrauigkeit Rv, gemessen gemäß der Beschreibung, von gleich oder weniger als 21,0 aufweist.

## Revendications

**1.** Joint se seringue (1) comprenant une section de contact de liquide (12) et

une section de glissement (13), dans lequel
un film inactif (15) est disposé en couches sur la section de contact de liquide (12),
aucun film inactif (15) n'est disposé en couches sur la section de glissement (13),
une chaîne de polymère modifié est formée sur une surface de la section de glissement (13),
la chaîne de polymère modifié est obtenue par l'addition d'un composé silane à une surface d'une chaîne de polymère et en outre en mettant à réagir un composé silane comprenant un groupe fluoroalkyle et un composé silane comprenant un groupe perfluoroéther ou en outre en mettant à réagir un composé silane comprenant un groupe fluoroalkyle,
un rapport en masse du composé silane comprenant le groupe fluroalkyle au composé silane comprenant le groupe perfluoroéther est de 60:40 à 100 :0, et
le composé silane comprenant le groupe fluoroalkyle est un composé représenté par la formule (1) suivante,

$$F_3C\text{-}(CF_2)_n\text{-}(CH_2)_m\text{-}Si(OR^1)_3 \qquad (1)$$

dans laquelle n vaut de 0 à 5, m vaut de 0 à 8 et $R^1$ représentent chacun un groupe alkyle et peuvent être identiques ou différents l'un de l'autre.

**2.** Joint de seringue (1) selon la revendication 1, dans lequel le film inactif (15) est formé à partir d'une résine à base d'oléfine et/ou d'une résine de fluor choisie dans le groupe constitué par un polymère tétrafluoroéthylène, un copolymère tétrafluoroéthylène-éthylène, un polymère tétrafluoroéthylène modifié, un copolymère tétrafluoroéthylène-éthylène modifié et un polymère chlorofluoroéthylène.

**3.** Joint de seringue (1) selon la revendication 1 ou 2, dans lequel la chaîne de polymère modifié est formée par un procédé de formation comprenant : une étape 1 de formation d'un point d'initiation de polymérisation sur la surface de la section de glissement (13) ; une étape 2 de polymérisation radicale d'un monomère débutant à partir du point d'initiation de polymérisation pour croître en une chaîne de polymère sur la surface de la section de glissement

(13) ; et une étape 3 d'addition du composé silane sur une surface de la chaîne de polymère et en outre en mettant à réagir le composé silane comprenant le groupe fluoroalkyle et/ou le composé silane comprenant le groupe perfluoroéther pour former la chaîne de polymère modifiée.

**4.** Joint de seringue (1) selon la revendication 1 ou 2, dans lequel la chaîne de polymère modifié est formée par un procédé de formation comprenant : une étape I de polymérisation radicale d'un monomère en présence d'un initiateur de photopolymérisation pour faire croître une chaîne de polymère sur la surface de la section de glissement (13) ; et une étape II d'addition du composé silane à une surface de la chaîne de polymère et en mettant à réagir en outre le composé silane comprenant le groupe fluoroalkyle et/ou le composé silane comprenant le groupe perfluoroéther pour former la chaîne de polymère modifié.

**5.** Joint de seringue (1) selon la revendication 3 ou 4, dans lequel le monomère est au moins un monomère choisi dans le groupe constitué par l'acide acrylique, les esters d'acide acrylique, les sels de métal alcalin d'acide acrylique, les sels d'amine d'acide acrylique, l'acrylamide, le diméthylacrylamide, le diéthylacrylamide, l'isopropylacrylamide, l'hydroxyéthylacrylamide, l'acryloylmorpholine, le méthoxyméthylacrylate, l'hydroxyéthylacrylate, l'acide méthacrylique, les esters d'acide méthacrylique, le sels de métal alcalin d'acide méthacrylique, les sels d'aminé d'acide méthacrylique, le méthacrylamide, le diméthylméthacrylamide, le diéthylméthacrylamide, l'isopropylméthacrylamide, l'hydroxyéthylméthacrylamide, la méthacryloylmorpholine, le méthoxyméthyl méthacrylate, l'hydroxyéthyl méthacrylate et l'acrylonitrile.

**6.** Joint de seringue selon l'une quelconque des revendications 1 à 5, dans lequel le composé silane comprenant le groupe perfluoroéther est un composé représenté par la formule (2) ou (3) suivante,

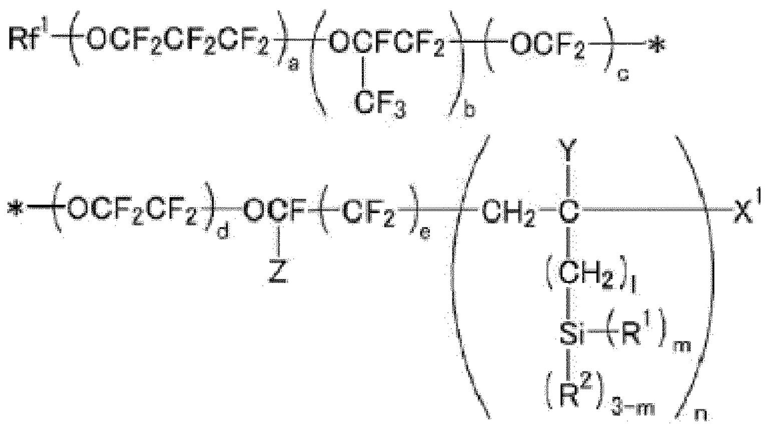

(2)

dans laquelle $Rf^1$ représente un groupe perfluoroalkyle ; Z représente un atome de fluor ou un groupe trifluorométhyle ; a, b, c, d et e sont identiques ou différents l'un de l'autre et chacun représente un nombre entier de 0 ou 1 ou plus, a+b+c+d+e vaut 1 ou plus, l'ordre existant de motifs répétés entre parenthèses par les indices a, b, c, d et e n'est pas limité à celui montré dans la formule ; Y représente un atome d'hydrogène ou un groupe alkyle en C1 à C4 ; $X^1$ représente un atome d'hydrogène, de brome ou d'iode ; $R^1$ représente un groupe hydroxyle ou un substituant hydrolysable ; $R^2$ représente un atome d'hydrogène ou un groupe hydrocarbure monovalent ; l représente 0, 1 ou 2 ; m représente 1, 2 ou 3 ; et n représente un nombre entier de 1 ou plus et les deux extrémités marquées par * sont directement liées l'une à l'autre,

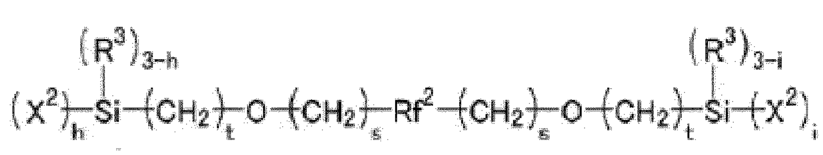

(3)

dans laquelle $Rf^2$ représente un groupe divalent qui contient un motif représenté par $-(C_kH_{2k})O-$ (k est un nombre entier de 1 à 6) et qui a une structure d'éther de perfluoropolyalkylène linéaire non ramifiée ; les $R^3$ sont identiques ou différents l'un de l'autre et chacun représente un groupe hydrocarbure monovalent en C1 à C8 ; $X^2$ sont identiques ou différents l'un de l'autre et chacun représente un groupe hydrolysable ou un atome d'halogène ; chaque s est identique ou différent l'un de l'autre et représente un nombre entier de 0 à 2 ; chaque t est identique ou différent

l'un de l'autre et représente un nombre entier de 1 à 5 ; et h et i sont identiques ou différents l'un de l'autre et chacun représente 1, 2 ou 3.

**7.** Joint de seringue (1) selon l'une quelconque des revendications 1 à 6, dans lequel la chaîne de polymère modifié a une longueur de 200 à 7 000 nm, telle que mesurée selon la description.

**8.** Joint de seringue (1) selon l'une quelconque des revendications 1 à 7, dans lequel

la chaîne de polymère modifié est fixée à au moins une partie d'une surface d'un joint de base (11),
la section de glissement (13) a une pluralité de protrusions annulaires (17a, 17b, 17c) sur une de ses surface de glissement, et
le joint de base (11) a une rugosité de surface Ra, telle que mesurée selon la description, inférieure ou égale à 1,0 à une première protrusion (17a) la plus proche d'une surface de partie supérieure parmi les protrusions annulaires (17a, 17b, 17c).

**9.** Joint de seringue (1) selon la revendication 8, dans lequel la rugosité de surface Ra du joint de base (11) est inférieure ou égale à 0,8.

**10.** Joint de seringue (1) selon la revendication 8, dans lequel la rugosité de surface Ra du joint de base (11) est inférieure ou égale à 0,6.

**11.** Joint de seringue (1) selon l'une quelconque des revendications 1 à 10, dans lequel

la section de glissement (13) a une pluralité de protrusions annulaires (17a, 17b, 17c) sur une de ses surfaces de glissement, et
une première protrusion (17a) la plus proche d'une surface de partie supérieure parmi les protrusions annulaires (17a, 17b, 17c) a une rugosité de surface Ra, telle que mesurée selon la description, inférieure ou égale à 1,0.

**12.** Joint de seringue (1) selon la revendication 11, dans lequel la rugosité de surface Ra est inférieure ou égale à 0,8.

**13.** Joint d seringue (1) selon la revendication 11, dans lequel la rugosité de surface Ra est inférieure ou égale à 0,6.

**14.** Joint de seringue (1) selon l'une quelconque des revendications 1 à 13, dans lequel

la section de glissement (13) a une pluralité de protrusions annulaires (17a, 17b, 17c) sur une de ses surfaces de glissement, et
une première protrusion (17a) la plus proche d'une surface de partie supérieure parmi les protrusions annulaires (17a, 17b, 17c) a une rugosité de surface Rz, telle que mesurée selon la description, inférieure ou égale à 25,0.

**15.** Joint de seringue (1) selon l'une quelconque des revendications 1 à 14, dans lequel

la section de glissement (13) a une pluralité de protrusions annulaires (17a, 17b, 17c) sur une de ses surfaces de glissement, et
une première protrusion (17a) la plus proche d'une surface de partie supérieure parmi les protrusions annulaires (17a, 17b, 17c) a une rugosité de surface Rv, telle que mesurée selon la description inférieure ou égale à 21,0.

**Fig. 1**

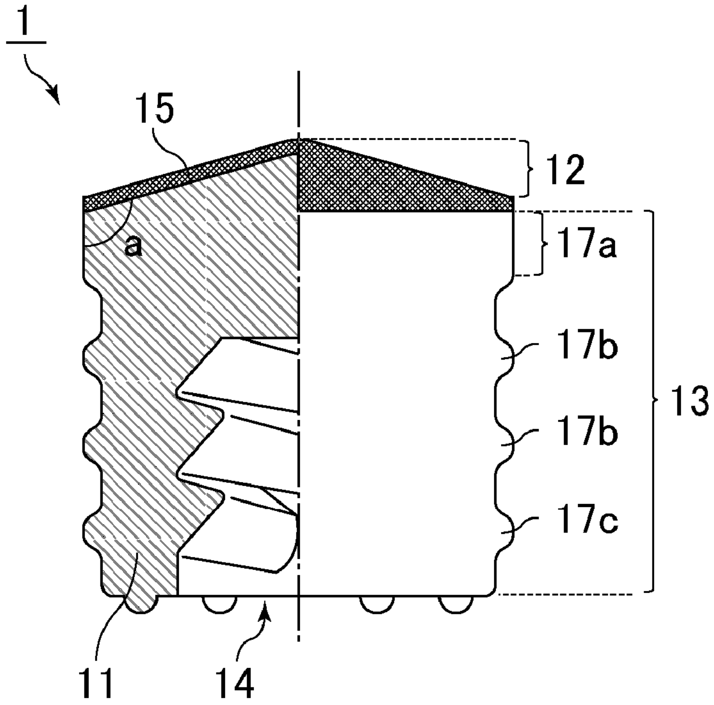

## REFERENCES CITED IN THE DESCRIPTION

*This list of references cited by the applicant is for the reader’s convenience only. It does not form part of the European patent document. Even though great care has been taken in compiling the references, errors or omissions cannot be excluded and the EPO disclaims all liability in this regard.*

### Patent documents cited in the description


- JP 2004298220 A
- JP 2010142537 A
- JP 2015043827 A
- EP 3281661 A1
- EP 3553116 A1
- EP 3639863 A1
- JP 2012147859 A